# FASCICULE DE BREVET EUROPEEN

(11) **EP 2 736 638 B1**
(45) Date de publication et mention de la délivrance du brevet: **20.04.2016**
(21) Numéro de dépôt: 12743512.1
(22) Date de dépôt: 12.07.2012
(51) Int. Cl.: B67D 7/44, C12M 1/12, B01L 1/02

(54) **CONTENEUR POUR LE TRANSFERT ASEPTIQUE D'UN PRODUIT BIOPHARMACEUTIQUE**
BEHÄLTER ZUM ASEPTISCHEN TRANSFER VON BIOPHARMAZEUTISCHEN PRODUKTEN
CONTAINER FOR THE ASEPTIC TRANSFER OF A BIOPHARMACEUTIC PRODUCT

(30) Priorité: 29.07.2011 FR 1157008
(43) Date de publication de la demande: 04.06.2014
(73) Titulaire: SARTORIUS STEDIM FMT SAS, 13400 Aubagne (FR)
(72) Inventeur: NODIN, Gaëlle, F-83470 Saint Maximin La Sainte Baume (FR)
(74) Mandataire: Derambure Conseil
(86) Numéro de dépôt international: PCT/FR2012/051665
(87) Numéro de publication internationale: WO 2013/017766

(56) Documents cités:
- EP-A1- 0 688 020
- WO-A1-2010/054031
- FR-A1- 2 872 446
- GB-A- 2 102 719
- GB-A- 2 218 663

## Description

L'invention est relative au domaine du transfert aseptique de produits biopharmaceutiques entre un conteneur et une enceinte close.

Plus précisément, l'invention concerne, selon un premier aspect, un conteneur spécialement destiné à assurer le transport et le transfert aseptique d'un produit biopharmaceutique depuis ou vers une enceinte close. L'invention concerne également, selon un deuxième aspect, un ensemble comprenant un tel conteneur, une enceinte close et un dispositif de jonction étanche permettant d'assurer le transfert aseptique de produits biopharmaceutiques entre le conteneur et l'enceinte close. L'invention concerne en outre, selon un troisième aspect, un procédé de transfert aseptique d'un produit biopharmaceutique entre le conteneur et l'enceinte close.

Il convient d'entendre ici par produit biopharmaceutique ou produit de biopharmacie, ce qui est relatif à la biotechnologie, à la pharmacie et plus généralement au domaine médical. En particulier, un produit biopharmaceutique est un produit issu de la biotechnologie - milieux de cultures, cultures cellulaires, solutions tampon, liquides de nutrition artificielle - ou un produit destiné à être utilisé dans le domaine pharmaceutique ou médical, au moins pour partie, sous forme solide plus ou moins finement divisé, sous forme liquide, ou sous forme pâteuse ou encore, plus généralement, un produit matériel - bouchon, récipient, tube ou ports intégrés, seringue, piston de seringue, moyen fonctionnel de traitement ou de conditionnement, ensemble plus ou moins complexe comprenant une pluralité de produits, etc. - destiné à être utilisé à l'intérieur de l'enceinte close.

Par convention, les termes « conteneur » ou « contenant » désignent ce qui, en biopharmacie, est apte et destiné dans son espace intérieur, à contenir, enfermer ou renfermer un certain contenu biopharmaceutique ou le cas échéant plusieurs contenus biopharmaceutiques, de façon statique plus ou moins durable ou pérenne. Un tel contenu biopharmaceutique est typiquement constitué par un ou plusieurs produit(s) biopharmaceutique(s) tel(s) que défini(s) précédemment. De tels conteneurs peuvent être rigides ou souples, à usage unique ou bien réutilisables, de tailles variées et sont par exemple des poches, des gaines, des conteneurs, des récipients, des bioréacteurs ou encore des goulottes à usage biopharmaceutique, cette liste n'étant pas limitative.

Il existe dans le domaine du transfert aseptique de produits biopharmaceutiques le besoin de faire communiquer un conteneur et une enceinte étanche, notamment pour des transferts de produits biopharmaceutiques, sans que l'étanchéité par rapport au milieu extérieur du conteneur et/ou de l'enceinte ne soit rompue et n'entraîne une contamination des produits biopharmaceutiques.

Pour ce faire, il est connu de l'état de la technique le document EP-A1-0 688 020 - conforme au préambule de la revendication 1 - qui décrit un conteneur comprenant une bride annulaire définissant une ouverture, un couvercle amovible susceptible d'obturer hermétiquement la bride et des moyens embarqués de verrouillage/déverrouillage du couvercle amovible contre la bride commandés par des moyens d'actionnement portés sur un dispositif de jonction étanche qui lui est associé.

Plus précisément, les moyens embarqués de verrouillage/déverrouillage comprennent, en premier lieu, un premier agencement formé par un logement traversant la bride et un logement borgne s'étendant à l'intérieur du couvercle amovible et dans le prolongement du logement traversant ainsi qu'une goupille interne et une goupille externe engagée dans le prolongement l'une de l'autre, dans ces deux logements. De cette façon, le conteneur peut passer d'une position de verrouillage initial dans laquelle la goupille interne est engagée à la fois dans la bride et dans le couvercle amovible, à une position de déverrouillage intermédiaire dans laquelle les extrémités des goupilles internes et externes se faisant face se placent à l'interface de la bride et du couvercle amovible, ce qui permet de désolidariser le couvercle amovible de la bride.

Les moyens embarqués de verrouillage/déverrouillage comprennent, en deuxième lieu, un deuxième agencement structurellement et fonctionnellement indépendant du premier, constitué par un autre logement traversant la bride, un autre logement borgne réalisé à l'intérieur du couvercle amovible et dans le prolongement du logement traversant ainsi qu'une goupille engagée dans le logement traversant. La goupille en question présente une longueur supérieure à celui du logement traversant de manière à pouvoir être déplacée jusqu'à l'intérieur du logement borgne pour verrouiller de manière irréversible le couvercle amovible sur la bride annulaire.

Cette solution technique répond au problème général précité dans la mesure où, associée au dispositif de jonction correspondant, elle permet d'assurer le transfert aseptique de produits biopharmaceutiques entre l'enceinte close et le conteneur. Cependant, cette réalisation soulève plusieurs inconvénients.

D'abord, la réalisation de deux agencements distincts comprenant, chacun, un logement traversant la bride annulaire pour passer, d'une part, en position de déverrouillage intermédiaire et, d'autre part, en position de verrouillage final implique une multiplication des zones à risques. En effet, par nature les logements traversant la bride annulaire peuvent se trouver en contact avec l'environnement extérieur à l'enceinte, d'une part, et l'environnement intérieur à l'enceinte, d'autre part. Dès lors, en cas de défaut de fabrication ou de dimensionnement des logements traversant ou des goupilles associées, il peut apparaître une perte d'intégrité susceptible de contaminer les produits biopharmaceutiques et/ou l'environnement intérieur de l'enceinte close. Il en résulte un défaut de fiabilité qui est, certes, limité mais qui ne peut cependant pas être négligé compte tenu des contraintes relatives au domaine de la biopharmacie.

En outre, cette multiplication des logements traversant la bride augmente l'encombrement au sein de la structure du conteneur, génère des coûts de production supplémentaires et complexifie l'évolution des systèmes de manipulation vers des solutions automatisées qui permettrait d'améliorer le degré de fiabilité de ces systèmes.

D'autre part, cette solution technique trouvait sa justification du fait de l'utilisation d'un dispositif de jonction doté d'un levier de manipulation manuel à deux sens de rotation distincts pour la mise en position de déverrouillage intermédiaire, d'une part, et la mise en position de verrouillage final, d'autre part. À l'origine, une telle solution technique facilitait les opérations de manutention des opérateurs puisqu'elle leur permettait de déplacer, d'abord, le levier dans un sens jusqu'à une position de butée pour placer le conteneur en position de déverrouillage intermédiaire puis de le déplacer, ensuite, dans l'autre sens, jusqu'à une autre position de butée afin d'atteindre la position de verrouillage finale. Toutefois, il est apparu à l'usage que cette succession d'opérations, combinée à l'utilisation d'un système de bridage indépendant, était complexe et finalement peu intuitif et donc source d'erreurs.

Il est également connu de l'état de la technique le document EP-A1-1 141 974 se rapportant à un conteneur comprenant des moyens embarqués de verrouillage/déverrouillage du couvercle amovible qui, comme l'indique expressément ce document, se trouvent être similaires à ceux divulgués dans le document FR-A-2 721 289.

En premier lieu, les moyens embarqués de verrouillage/déverrouillage assurent le déverrouillage du couvercle amovible du conteneur par enfoncement de deux goupilles dans des logements coaxiaux réalisés dans la bride et le couvercle amovible, jusqu'à ce que la zone de contact entre ces goupilles soit amené au niveau de la frontière entre le logement traversant aménagé dans la bride et le logement borgne réalisé dans le couvercle amovible. En deuxième lieu, ces moyens embarqués de verrouillage/déverrouillage procèdent au verrouillage du couvercle amovible sur le conteneur par enfoncement d'une autre goupille dans d'autres logements coaxiaux réalisés dans la bride et le couvercle amovible.

Il découle de cette similitude que les problèmes techniques précédemment évoqués se posent de façon identique dans le cadre de la réalisation et de l'utilisation de ce conteneur sans qu'il soit nécessaire de les expliciter à nouveau.

Il est, par ailleurs, connu de l'art antérieur le document WO-A1-2010/054031 qui décrit un conteneur comprenant une bride annulaire définissant une ouverture, un couvercle amovible susceptible d'obturer hermétiquement la bride et des moyens embarqués de verrouillage/déverrouillage du couvercle amovible contre la bride commandés par des moyens d'actionnement réversibles portés directement sur le conteneur.

Plus particulièrement, les moyens embarqués de verrouillage/déverrouillage sont formés par plusieurs agencements comprenant, chacun, un logement traversant la bride, un logement borgne réalisé dans le couvercle amovible, un pion apte à pénétrer dans le logement traversant la bride et dans le logement borgne du couvercle amovible ainsi qu'un levier capable de commander de manière réversible le déplacement du pion. Le conteneur est ainsi capable de présenter deux positions alternatives seulement. Dans la première position, dite position de verrouillage du couvercle amovible contre la bride, le levier est relevé de sorte que le pion pénètre simultanément dans le logement traversant la bride et dans le logement borgne du couvercle amovible et que le couvercle amovible se trouve verrouiller hermétiquement contre la bride. Dans la deuxième position, dite position de déverrouillage du couvercle amovible, le levier est abaissé de sorte que le pion ne pénètre plus que dans le logement traversant la bride permettant ainsi de libérer le couvercle amovible par rapport à cette bride.

Cette solution technique se distingue de celles évoquées dans les documents EP-A1-1 141 974 et FR-A-2 721 289 en ce qu'elle peut permettre aux opérateurs de faire passer, eux-mêmes, le conteneur d'une position de verrouillage à une position de déverrouillage de façon réversible et par la seule manipulation des leviers agencés sur la bride du conteneur. Le conteneur est ainsi réutilisable.

Cet agencement présente toutefois également des inconvénients.

Du fait de la réversibilité du verrouillage/déverrouillage, d'une part, et de l'accessibilité des moyens d'actionnement - à savoir les leviers - portés directement sur la bride du conteneur, d'autre part, les risques d'ouverture intentionnelle ou inopinée de conteneurs emplis de produits biopharmaceutiques sont considérablement accrus. Il est d'ailleurs possible que de tels conteneurs soient d'abord ouverts, rompant ainsi l'isolation des produits biopharmaceutiques par rapport à l'extérieur, puis qu'ils soient ensuite refermés de sorte que cette rupture de confinement ne puisse pas être identifiée.

Le document WO2010/054031 divulgue un conteneur destiné à assurer le transfert aseptique d'un produit vers une enceinte. Le conteneur comprend une bride annulaire définissant une ouverture et il est prévu un couvercle amovible.

Le document GB 2 102 719 divulgue un système pour faire passer des matières dangereuses dans et hors d'une enceinte, telle qu'une boîte à gants, à travers un orifice dans une paroi de l'enceinteL'orifice est normalement fermé par une porte, qui coopère avec une fermeture d'extrémité amovible sur un récipient ou analogue lorsque celui-ci est présenté à et fixé à l'orifice. Le récipient est fixé en position à l'orifice au moyen d'une bague d'accouplement rotativeUn dispositif de verrouillage assure que la porte ne peut pas être ouverte en l'absence d'un conteneur dans le port et que le conteneur ne peut pas être retiré du port quand la porte est ouverte. En lieu et place du conteneur, on peut utiliser un gant solidaire d'un manchon rigide pour permettre à l'opérateur d'effectuer une fonction de travail au sein de la boîte à gants.

Le document FR 2 872 446 divulgue un dispositif de transfert étanche à double porte pour assurer un transfert étanche entre une première enceinte étanche, par exemple une cellule de confinement et une seconde enceinte étanche, par exemple une boîte de transfert, comprenant deux portes équipées chacune de moyen de verrouillage pour la verrouiller sur une bride comportant une ouverture centrale et d'un organe d'actionnement pour actionner les moyens de verrouillage, dans lequel les organes d'actionnement sont montés tournant dans les portes et comportent un hublot.

Le document EP 0 688 020 divulgue un dispositif de jonction étanche entre deux enceintes isolées d'un milieu extérieur et munies, chacune, d'une porte apte à obturer hermétiquement une ouverture de l'enceinte correspondante délimitée par une bride annulaire, chaque porte étant définie avec une face interne au contact de l'intérieur de l'enceinte et avec une face externe au contact dudit milieu extérieur et lesdites portes étant pourvues de moyens d'assemblage coopérants permettant de les appliquer l'une contre l'autre de façon hermétique afin d'isoler mutuellement leurs faces externes, dans lequel l'une des deux enceintes est du type à usage unique et comporte des moyens de verrouillage final de sa propre porte, commandés par des premiers moyens d'actionnement installés sur l'autre enceinte.

Le document G8 2 218 663 divulgue un système à double couvercle comprenant un premier récipient cylindrique, ouvert à une extrémité, un premier couvercle pour le premier récipient, des moyens définissant un orifice pour un second récipient, et un second couvercle pour le dit orifice, le premier récipient ayant un joint périphérique d'étanchéité des moyens pour le port et le premier couvercle, et le second couvercle ayant un joint périphérique d'étanchéité à l'orifice des moyens et au premier couvercle, dans lequel le premier couvercle comprend un mécanisme de capture d'un premier élément rotatif pour fixer le premier couvercle au premier récipient, le premier récipient comprenant des moyens avec lesquels le mécanisme de capture du premier élément rotatif peut s'engager, le second couvercle incorpore un arbre rotatif s'étendant à travers lui, et il est prévu des moyens pour faire tourner l'arbre, et un mécanisme de capture du second organe rotatif qui peut être actionné par l'arbre pour fixer le premier couvercle au second couvercle, le premier couvercle comportant des moyens avec lesquels le mécanisme de capture du second organe rotatif peut s'engager, le système incorporant enfin un mécanisme à dents de crabotage avec au moins vingt dents qui s'engage lorsque le premier couvercle est adjacent au deuxième couvercle de telle sorte que la rotation de l'arbre entraîne la rotation du premier et du second mécanismes de capture rotatifs simultanément.

Dans ce contexte, la présente invention a pour but de proposer un conteneur spécialement destiné à assurer le transport et le transfert aseptique d'un produit appartenant au domaine biopharmaceutique qui soit exempt de l'une au moins des limitations précédemment évoquées.

Plus particulièrement, la présente invention se rapporte à un conteneur capable d'assurer un tel transfert sans rupture d'étanchéité, qui présente un nombre de zones à risque limité, qui soit simple à produire et qui permette une utilisation intuitive de manière à limiter les risques de contamination inopinée ou intentionnelle.

À cet égard, l'invention concerne, selon un premier aspect, un conteneur spécialement destiné à assurer le transport et le transfert aseptique d'un produit biopharmaceutique depuis ou vers une enceinte close dotée d'un dispositif de jonction étanche, comprenant : une bride annulaire délimitant une ouverture ; un couvercle amovible apte à obturer hermétiquement l'ouverture de la bride annulaire ; des moyens embarqués de verrouillage/déverrouillage du couvercle amovible sur la bride annulaire ; et une enveloppe périphérique solidaire de la bride annulaire et délimitant un espace intérieur confiné apte à recevoir les produits appartenant au domaine biopharmaceutique ; dans lequel les moyens embarqués de verrouillage/déverrouillage comprennent au moins un agencement fonctionnel embarqué de verrouillage/déverrouillage formé, d'une part, par un logement traversant formé dans la bride annulaire et un logement borgne formé dans le couvercle amovible et dans le prolongement du logement traversant lorsque le couvercle amovible obture hermétiquement l'ouverture de la bride annulaire et, d'autre part, par une goupille à positionnement radial interne et une goupille à positionnement radial externe susceptibles d'être introduites et de se déplacer à l'intérieur du logement borgne du couvercle amovible et du logement traversant de la bride annulaire ; le conteneur étant apte à se trouver dans une position de verrouillage initial dans laquelle, d'une part, la goupille à positionnement radial interne présente une portion fonctionnelle interne de verrouillage initial agencée dans le logement borgne du couvercle amovible et une portion fonctionnelle externe de verrouillage initial agencée dans le logement traversant de la bride annulaire de manière à interdire le déplacement relatif du couvercle amovible vis-à-vis de la bride annulaire et, d'autre part, la goupille à positionnement radial externe est au moins partiellement agencée dans le logement traversant de la bride annulaire ; le conteneur étant apte à se trouver dans une position de déverrouillage intermédiaire dans laquelle, d'une part, la goupille à positionnement radial interne est au moins partiellement agencée dans le logement borgne du couvercle amovible et entièrement exclue du logement traversant de la bride annulaire et, d'autre part, la goupille à positionnement radial externe est au moins partiellement agencée dans le logement traversant de la bride annulaire et entièrement exclue du logement borgne du couvercle amovible de sorte à autoriser le déplacement le relatif du couvercle amovible vis-à-vis de la bride annulaire. Plus particulièrement, le conteneur se caractérise en ce qu'il est apte à se trouver dans une position de verrouillage final dans laquelle, d'une part, la goupille à positionnement radial interne est agencée dans le logement borgne du couvercle amovible et, d'autre part, la goupille à positionnement radial externe présente une portion fonctionnelle interne de verrouillage final agencée dans le logement borgne du couvercle amovible et une portion fonctionnelle externe de verrouillage final agencée dans le logement traversant de la bride annulaire de manière à interdire le déplacement relatif du couvercle amovible vis-à-vis de la bride annulaire.

Une telle réalisation permet d'éviter l'utilisation de deux logements distincts, dans la bride annulaire et dans le couvercle amovible, pour réaliser le passage en position de déverrouillage intermédiaire, d'une part, et le passage en position de verrouillage final, d'autre part. De cette façon, les risques de fuites susceptibles de provenir de défaut de fabrication ou de dimensionnement sont limités. En outre, l'utilisation d'agencements fonctionnels embarqués de verrouillage/déverrouillage ne nécessitant de déplacer les goupilles que selon une seule direction et un seul sens de déplacement simplifie le mécanisme d'actionnement des moyens embarqués de verrouillage/déverrouillage, le rend plus intuitif à utiliser et autorise dans le même temps une automatisation de cette procédure.

Selon une réalisation, le logement traversant et la goupille à positionnement radial externe présentent des longueurs telles que, lorsque les moyens embarqués de verrouillage/déverrouillage sont dans la position de verrouillage initial, ladite goupille à positionnement radial externe présente une partie extrême fonctionnelle à positionnement radial externe soit exclue du logement traversant, soit logée à l'intérieur du logement traversant, soit affleurant l'extrémité externe débouchant du logement traversant.

Selon une réalisation également, le logement traversant et la goupille à positionnement radial externe présentent des longueurs telles que, lorsque les moyens embarqués de verrouillage/déverrouillage sont dans la position de déverrouillage intermédiaire, ladite goupille à positionnement radial externe présente une partie extrême fonctionnelle à positionnement radial externe soit exclue du logement traversant, soit logée à l'intérieur du logement traversant, soit affleurant l'extrémité externe débouchant du logement traversant.

Selon une réalisation, en outre, le logement traversant et la goupille à positionnement radial externe présentent des longueurs telles que, lorsque les moyens embarqués de verrouillage/déverrouillage sont dans la position de verrouillage final, ladite goupille à positionnement radial externe présente une partie extrême fonctionnelle à positionnement radial externe soit exclue du logement traversant, soit logée à l'intérieur du logement traversant, soit affleurant l'extrémité externe débouchant du logement traversant.

Le fait que la goupille à positionnement radial externe soit exclue du logement traversant lorsque le conteneur est en position de verrouillage initial ou en position de déverrouillage intermédiaire permet de faciliter l'opération des moyens stationnaires de verrouillage/déverrouillage consistant à faire passer le conteneur de la position de verrouillage initial à la position de déverrouillage intermédiaire ou bien de la position de déverrouillage intermédiaire à la position de verrouillage final. En effet, la goupille à positionnement radial externe reste ainsi accessible sans que l'élément poussoir à déplacement radial n'ait à se déplacer jusqu'à l'intérieur du logement traversant. En outre, le fait que cette goupille à positionnement radial externe soit également exclue du logement traversant lorsque le conteneur est en position de verrouillage final peut être utile lorsqu'il est nécessaire de procéder à un démontage ou bien à une réouverture du conteneur après sa mise en position de verrouillage finale car la goupille à positionnement radial externe est alors plus accessible que si elle était positionnée entièrement dans le logement traversant de la bride annulaire.

Au contraire, le fait que la goupille à positionnement radial externe soit entièrement positionnée à l'intérieur du logement traversant lorsque le conteneur est en position de verrouillage initial, en position de déverrouillage intermédiaire ou bien en position de verrouillage final permet limiter l'accès à cette goupille à positionnement radial externe et rend donc moins probable et plus complexe l'actionnement inopiné des moyens embarqués de verrouillage/déverrouillage. En outre, le passage en position de verrouillage final devient alors quasiment irréversible. Cette option est donc avantageuse, notamment lorsque le conteneur est à usage unique.

En outre, utiliser la goupille à positionnement radial externe de sorte qu'elle affleure l'extrémité externe débouchant du logement traversant - dans l'une ou l'autre des positions de verrouillage initiale, de déverrouillage intermédiaire ou de verrouillage final - permet de limiter l'introduction des moyens stationnaires de déverrouillage ou de verrouillage en profondeur à l'intérieur du logement traversant tout en limitant l'accès à cette goupille à positionnement radial externe, ce qui atténue les risques d'ouverture inopinée du conteneur.

Selon une réalisation, la goupille à positionnement radial interne et la goupille à positionnement radial externe présentent des longueurs sensiblement identiques. Cela permet de simplifier l'approvisionnement, la production et l'assemblage des goupilles à positionnement radial interne et externe puisqu'il n'est alors pas nécessaire de distinguer les goupilles eu égard à leur dimensions respectives.

Selon une réalisation, le logement traversant, le logement borgne, la goupille à positionnement radial interne et la goupille à positionnement radial externe sont coaxiaux et orientés dans une direction radiale relative à la bride annulaire.

Selon une réalisation alternative, le logement borgne, la goupille à positionnement radial interne et la goupille à positionnement radial externe sont coaxiaux et orientés dans une direction formant un angle α par rapport à une direction radiale relative à la bride annulaire.

Par ailleurs, selon une réalisation, le conteneur comprend également des moyens embarqués de cloisonnement et de protection agencés sur un pourtour périphérique externe de la bride annulaire de manière à faire obstacle à la manipulation inopinée des moyens embarqués de verrouillage/déverrouillage. Ces moyens peuvent ainsi limiter les risques de manipulation inopinée, susceptibles de faire passer le conteneur de sa position de verrouillage initiale à la position de déverrouillage intermédiaire, notamment pendant son transport.

Dans ce cas, selon une réalisation, les moyens embarqués de cloisonnement et de protection comprennent au moins un agencement fonctionnel embarqué de cloisonnement et de protection disposé autour du logement traversant de manière à faire obstacle à la manipulation inopinée de la goupille à positionnement radial externe.

Alors, selon une réalisation, lequel l'agencement fonctionnel embarqué de cloisonnement et de protection présente des dimensions radiales telles que, en position de verrouillage provisoire, la goupille à positionnement radial externe ne s'étend pas au-delà desdits moyens de cloisonnement et de protection.

Selon une réalisation, l'agencement fonctionnel embarqué de cloisonnement et de protection comporte au moins un ergot, de préférence deux ergots, disposé(s) autour du logement traversant de la bride annulaire.

Selon une réalisation alternative ou complémentaire, l'agencement de cloisonnement et de protection peut également comporter un alésage formé dans la bride annulaire et présentant un diamètre supérieur au diamètre du logement traversant. Il est ainsi plus délicat d'accéder à la goupille à positionnement radial externe sans que cela complique pour autant le déplacement de cette dernière par les moyens stationnaires de déverrouillage et de verrouillage.

Selon une réalisation, le conteneur comprend également des moyens embarqués de bridage temporaire agencés sur un pourtour périphérique externe de la bride annulaire de manière à permettre le bridage temporaire du conteneur par blocage axial sur l'enceinte close via l'actionnement du dispositif de jonction étanche.

Dans ce cas, selon une réalisation, les moyens embarqués de bridage temporaire comprennent au moins un agencement fonctionnel embarqué de bridage temporaire formé par au moins un ergot, de préférence deux ergots, présentant une surface fonctionnelle embarquée de bridage axial apte à venir en appui contre une surface fonctionnelle stationnaire de bridage axial du dispositif de jonction étanche de manière à bloquer axialement le déplacement du conteneur vis-à-vis de l'enceinte close.

En outre, selon une réalisation, les moyens embarqués de bridage temporaire sont au moins en partie formés par les moyens de protection et de cloisonnement, ce qui permet d'alléger et de simplifier le conteneur tout en diminuant les coût de production.

Selon une réalisation, l'agencement fonctionnel embarqué de bridage temporaire et l'agencement fonctionnel embarqué de cloisonnement et de protection sont formés par au moins un ergot commun, et de préférence deux ergots communs.

Selon une réalisation, les moyens embarqués de verrouillage/déverrouillage comportent n agencements fonctionnels embarqués de verrouillage/déverrouillage régulièrement répartis autour de la bride annulaire et du couvercle amovible, avec n supérieur ou égal à 1. Ainsi le verrouillage initiale et final du couvercle amovible contre la bride annulaire est renforcé.

Dans ce cas, selon une réalisation, les moyens embarqués de cloisonnement et de protection comprennent n agencements fonctionnels embarqués de cloisonnement et de protection régulièrement répartis autour de la bride annulaire et indexés vis-à-vis des n agencements fonctionnels embarqués de verrouillage/déverrouillage.

Selon une réalisation, les moyens embarqués de bridage temporaire comprennent m agencements fonctionnels embarqués de bridage temporaire régulièrement répartis autour de la bride annulaire, avec m supérieur ou égal à 1.

Dans ce cas, selon une réalisation, n est égal à m et les agencements fonctionnels embarqués de bridage temporaire sont indexés vis-à-vis des agencements fonctionnels embarqués de verrouillage/déverrouillage.

Selon une réalisation, le conteneur est de type à usage unique.

L'invention concerne également, selon un deuxième aspect, un ensemble comprenant une enceinte, un dispositif de jonction étanche et un conteneur tel que décrit précédemment, en vue de réaliser le transfert aseptique d'un produit biopharmaceutique entre l'enceinte et le conteneur.

Selon une réalisation, l'enceinte comprend une paroi périphérique dans laquelle est aménagée une ouverture hermétique obturée par une porte amovible et dans lequel le dispositif de jonction étanche comprend : des moyens stationnaires de bridage temporaire aptes à maintenir le conteneur bridé contre l'enceinte de sorte que le couvercle amovible dudit conteneur se trouve appliqué hermétiquement contre la porte de ladite enceinte ; des moyens stationnaires de déverrouillage aptes à faire passer le conteneur d'une position de verrouillage initial dans laquelle le couvercle amovible obture hermétiquement le conteneur à une position de déverrouillage intermédiaire dans laquelle le couvercle amovible est désolidarisé du conteneur et maintenu hermétiquement contre la porte de l'enceinte de manière à assurer une communication aseptique entre ledit conteneur et ladite enceinte ; des moyens stationnaires de verrouillage aptes à faire passer le conteneur de la position de déverrouillage intermédiaire à une position de verrouillage final dans laquelle ledit couvercle amovible obture à nouveau de façon hermétique le conteneur ; une couronne fonctionnelle annulaire apte à se déplacer par rotation autour d'un axe géométrique de rotation de manière à actionner les moyens stationnaires de déverrouillage et les moyens stationnaires de verrouillage du conteneur ; les moyens stationnaires de bridage temporaire, les moyens stationnaires de déverrouillage et les moyens stationnaires de verrouillage étant liés mécaniquement à la couronne fonctionnelle annulaire de sorte que la rotation unidirectionnelle de ladite couronne fonctionnelle annulaire autour de l'axe géométrique de rotation entraîne successivement l'actionnement des moyens stationnaires de bridage temporaire assurant le maintien en position du conteneur contre l'enceinte, puis l'actionnement des moyens stationnaires de déverrouillage assurant le passage en position de déverrouillage intermédiaire du conteneur, puis l'actionnement des moyens stationnaires de verrouillage du conteneur assurant le passage en position de verrouillage final du conteneur, et l'actionnement des moyens stationnaires de bridage temporaire du conteneur assurant la libération du conteneur.

En outre, selon un troisième aspect, l'invention se rapporte à un procédé de transfert aseptique destiné à assurer le transfert aseptique d'un produit biopharmaceutique entre un conteneur et une enceinte appartenant à un ensemble tel que décrit précédemment et comprend des étapes successives consistant à : disposer de l'enceinte, du dispositif de jonction étanche et du conteneur ; positionner le conteneur contre la paroi périphérique de l'enceinte ; générer un bridage axial de la bride annulaire du conteneur contre la paroi périphérique de l'enceinte par rotation unidirectionnelle de la couronne fonctionnelle annulaire ; générer le passage du conteneur de la position de verrouillage initial à la position de déverrouillage intermédiaire par rotation unidirectionnelle de la couronne fonctionnelle annulaire entraînant le déplacement des goupilles à positionnement radial interne et externe à l'intérieur des logements traversant et borgne ; ouvrir simultanément la porte amovible de l'enceinte et le couvercle amovible du conteneur, le couvercle amovible étant fixé hermétiquement contre la porte amovible ; transférer de manière aseptique un ou plusieurs(s) produit(s) biopharmaceutique(s) entre le conteneur et l'enceinte; refermer simultanément la porte amovible de l'enceinte et le couvercle amovible du conteneur le couvercle amovible étant fixé hermétiquement contre la porte amovible ; générer le passage du conteneur par de la position de déverrouillage intermédiaire à la position de verrouillage final par rotation unidirectionnelle de la couronne fonctionnelle annulaire entraînant le déplacement des goupilles à positionnement radial interne et externe à l'intérieur des logements traversant et borgne ; générer le débridage axial de la bride annulaire du conteneur vis-à-vis de la paroi périphérique de l'enceinte par rotation unidirectionnelle de la couronne fonctionnelle annulaire.

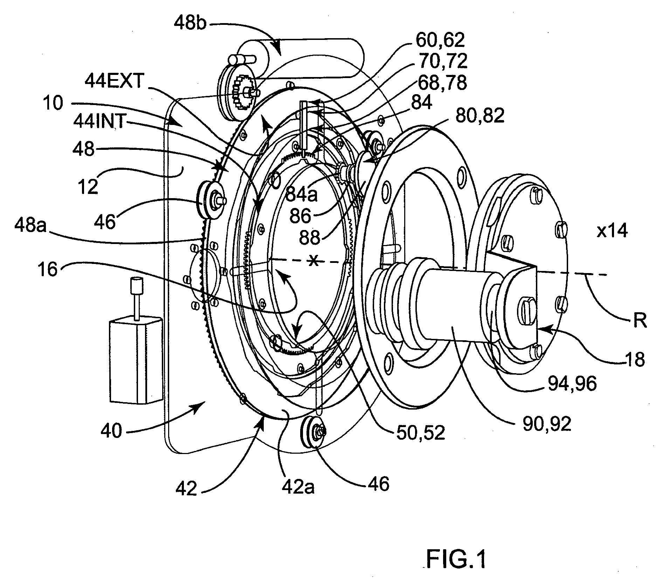

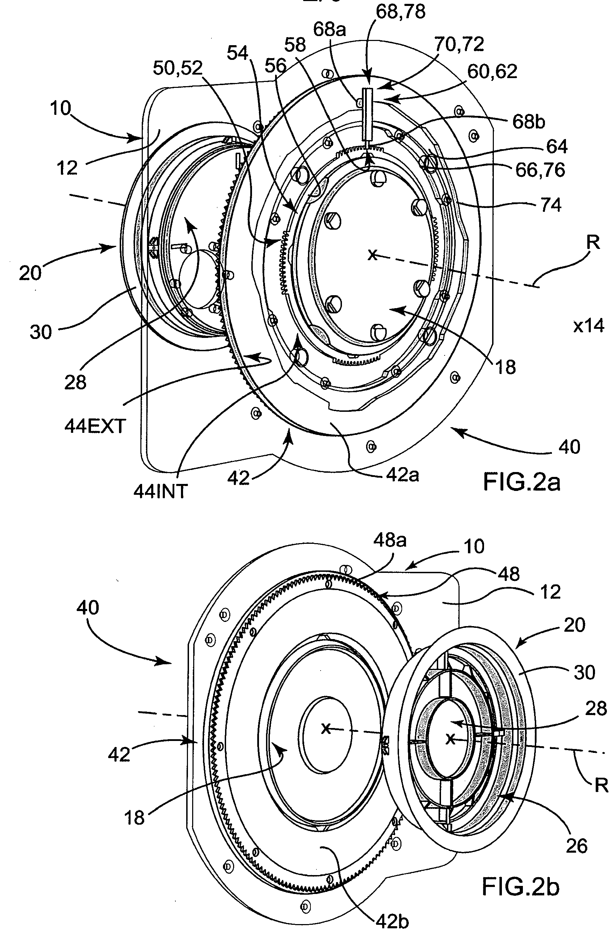

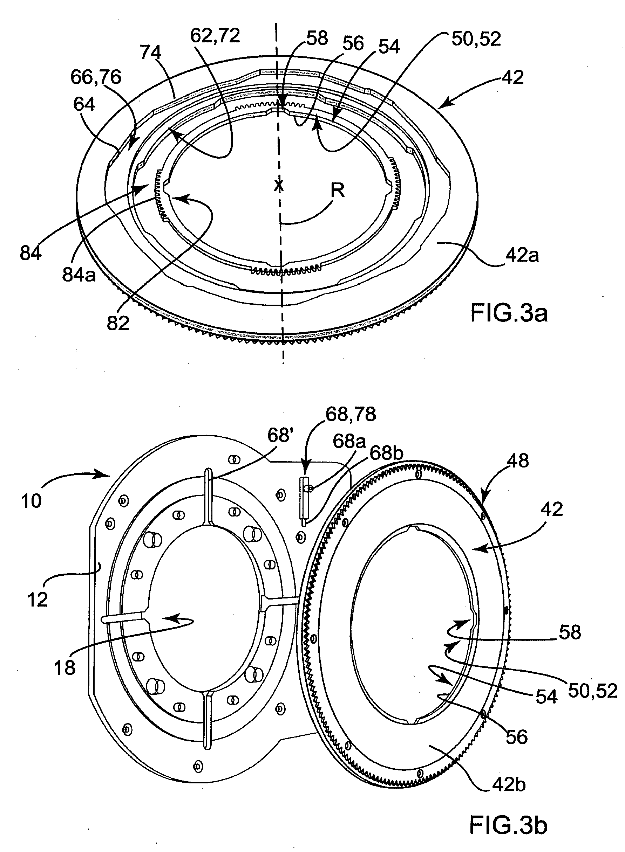

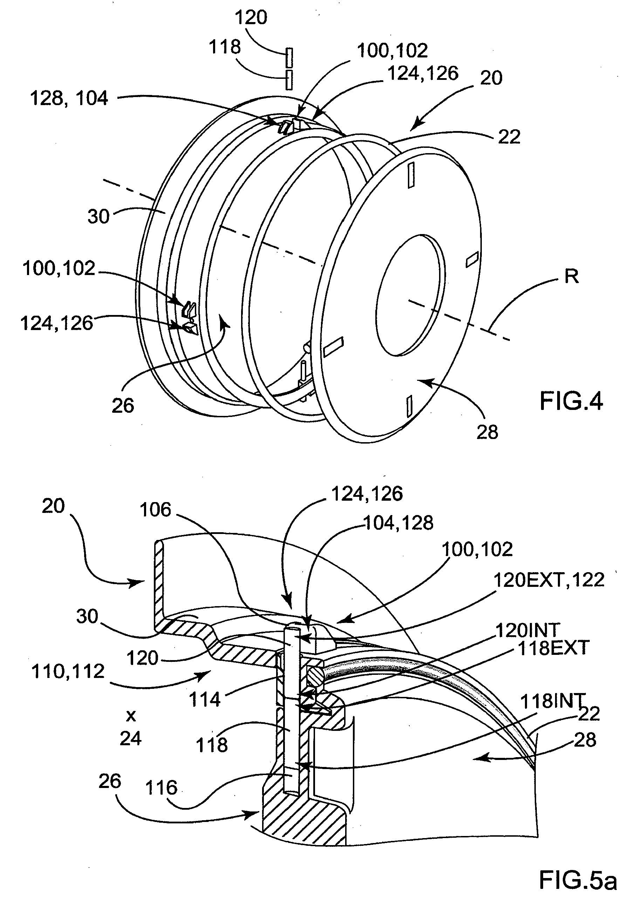

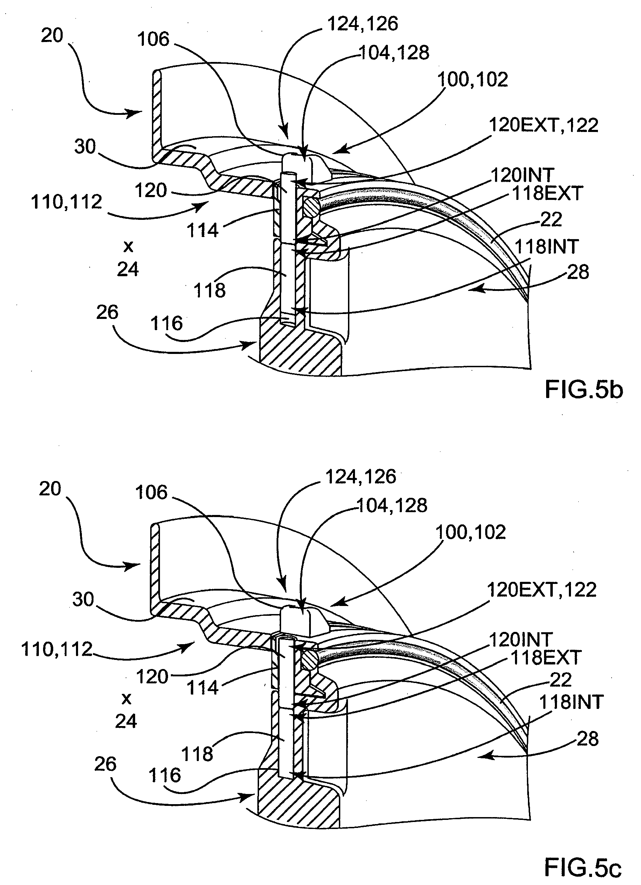

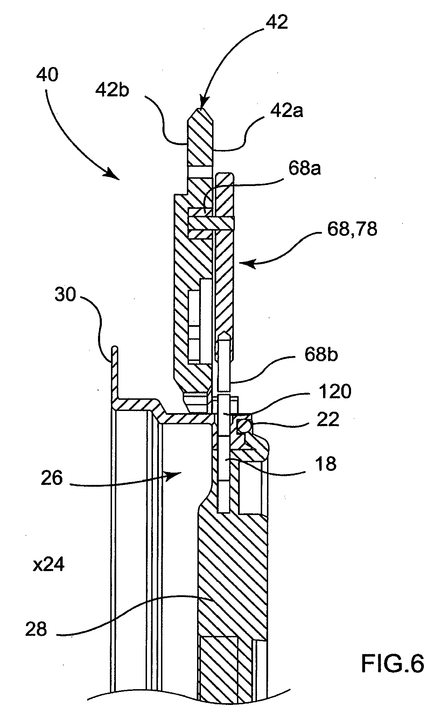

D'autres caractéristiques et avantages de l'invention ressortiront clairement de la description qui en est faite ci-après, à titre indicatif et nullement limitatif, en référence aux dessins annexés, dans lesquels :
▪ La figure 1 est une vue d'ensemble générale, en perspective et en vue éclatée, d'un mode de réalisation d'un dispositif de jonction étanche associé à la paroi périphérique d'une enceinte ;
▪ Les figures 2a et 2b sont deux vues d'ensemble générale, en perspective depuis l'intérieur de l'enceinte et depuis l'extérieur de l'enceinte, du dispositif de jonction étanche de la figure 1 installé sur une portion de la paroi périphérique de l'enceinte et prêt à être associé avec la bride annulaire d'un conteneur selon l'invention ;
▪ Les figures 3a et 3b sont deux vues de détail, en perspective, de la couronne fonctionnelle annulaire appartenant au mode de réalisation du dispositif de jonction de la figure 1 ;
▪ La figure 4 est une vue d'ensemble générale, en perspective et en vue éclatée, d'un mode de réalisation d'un conteneur selon l'invention dans laquelle l'enveloppe périphérique n'est pas représentée ;
▪ La figure 5a est une vue de détail, en coupe transversale, représentant une partie seulement du conteneur de la figure 4 en position de verrouillage initial ;
▪ La figure 5b est une vue de détail, en coupe transversale, représentant une partie seulement du conteneur de la figure 4 en position de déverrouillage intermédiaire ;
▪ La figure 5c est une vue de détail, en coupe transversale, représentant une partie seulement du conteneur de la figure 4 en position de verrouillage final ;
▪ La figure 6 est une représentation de détail, en coupe transversale, d'un ensemble comprenant une enceinte close, un dispositif de jonction étanche et un conteneur selon l'invention associé au dispositif de jonction étanche et en position de verrouillage initiale.

La figure 1 représente une enceinte 10 close comportant une paroi périphérique 12 définissant un espace intérieur 14 confiné et une ouverture circulaire 16 autorisant l'introduction de produits biopharmaceutiques (non représentés) dans l'espace intérieur 14 confiné.

L'enceinte 10 - qui peut être un local ou toute autre installation analogue - est conçue pour être en permanence isolée du milieu extérieur. Ainsi, pour éviter toute perte d'intégrité et conserver un environnement aseptique dans l'espace intérieur 14, l'ouverture annulaire 16 est obturée hermétiquement par une porte amovible 18 positionnée dans l'espace intérieur et capable de passer d'une position fermée dans laquelle l'ouverture circulaire 16 est obstruée à une position ouverte dans laquelle l'ouverture circulaire 16 n'est plus obstruée.

Par convention, les termes « interne » et « externe » qualifient dans la suite de ce document les positions relatives d'objets vis-à-vis de l'axe géométrique de l'ouverture annulaire 16. Ainsi l'objet qualifié d' « interne » ou ayant un « positionnement radial interne » doit-il être considéré comme placé au plus proche de l'axe géométrique de l'ouverture annulaire 16 tandis que l'objet qualifié d' « externe » ou ayant un positionnement radial « externe » doit, comparativement, être considéré comme placé au plus loin de l'axe géométrique de l'ouverture annulaire 16.

Les figure 2a et 2b représentent un conteneur 20 destiné à transporter des produits biopharmaceutiques et prêt à être associé à l'enceinte 10 close pour réaliser un transfert aseptique de produits biopharmaceutiques vers ou depuis cette enceinte 10.

Le conteneur 20 comporte, à cet égard, une enveloppe périphérique (non représentée) définissant un espace intérieur 24 confiné, dans lequel peuvent être introduits les produits biopharmaceutiques. L'enveloppe périphérique comporte également une ouverture annulaire 26 délimitée par une bride annulaire 30 sur laquelle est solidairement fixée l'enveloppe périphérique. Le conteneur 20 comporte, en outre, un couvercle amovible 28 capable d'obturer hermétiquement l'ouverture 24 de la bride annulaire 30 en reposant sur un joint d'étanchéité 22.

Le conteneur 20 peut être réalisé selon différents mode de réalisation et, à cet égard, présenter une enveloppe périphérique souple et destinée à un usage unique ou bien rigide et destinée à être réutilisée après une procédure de décontamination bien connue de l'homme du métier.

Les figures 1, 2a et 2b illustrent un mode de réalisation d'un dispositif de jonction étanche 40.

Ce dispositif de jonction étanche 40 est destiné à permettre le transfert aseptique de produits biopharmaceutiques provenant du conteneur 20 (décrit ultérieurement) vers l'enceinte 10. Le dispositif de jonction étanche 40 a donc pour objet, d'abord, de permettre l'assemblage de l'enceinte 10 et du conteneur 20, ensuite, d'autoriser la mise en communication temporaire de l'espace intérieur 14 de cette enceinte 10 avec l'espace intérieur de ce conteneur 20 pour assurer le transfert de produits biopharmaceutiques de l'un vers l'autre et, enfin, d'assurer la séparation de ladite enceinte 10 et dudit conteneur 20.

Ces étapes successives - englobées par la suite sous la qualification de « transfert aseptique » - doivent être réalisées sans qu'il y ait de communication entre le milieu extérieur, d'une part, et les espaces intérieur 14, 24 de l'enceinte 10 et du conteneur 20, d'autre part.

Pour ce faire, le dispositif de jonction étanche 40 selon le mode de réalisation de la figure 1 présente une partie formée à l'extérieure de l'enceinte 10 et supportée par la paroi périphérique12 - ou par une pièce de support distincte de la paroi périphérique 12 mais fixée à demeure sur elle - en regard de l'ouverture annulaire 16. Plus précisément, le dispositif de jonction étanche 40 comporte une couronne fonctionnelle annulaire 42 dotée d'une face 42a orientée vers la paroi périphérique 12 de l'enceinte 10 et d'une face 42b orientée vers le milieu extérieur.

La couronne fonctionnelle annulaire 42 présente également un pourtour périphérique interne 44_{INT} et un pourtour périphérique externe 44_{EXT} inscrit à l'intérieur de trois galets porteurs 46 eux-mêmes su pportés par la face extérieure de la paroi périphérique 12. De cette façon, la couronne fonctionnelle annulaire 42 est positionnée à l'extérieur de l'enceinte 10, ce qui facilite les éventuelles opérations de maintenance. Les trois galets porteurs 46 forment, d'une part, un support pour la couronne fonctionnelle annulaire et permettent, d'autre part, sa rotation autour de l'axe géométrique de l'ouverture annulaire 16. Il convient cependant de souligner que le maintien en position de la couronne annulaire fonctionnelle pourrait être assuré par tout autre élément mécanique analogue (roulement, pa lier, etc.).

Selon l'exemple de réalisation de la figure 1, la couronne fonctionnelle annulaire 42 qui présente plusieurs portions ayant des fonctions mécaniques distinctes (décrites ultérieurement) est réalisée d'un seul tenant - par usinage, moulage, ou analogue - à partir d'un seul et unique bloc de matière. Cette réalisation présente l'avantage de simplifier la production de la couronne fonctionnelle annulaire, de diminuer dans le même temps les coûts de production et d'assurer un positionnement optimal des surfaces fonctionnelles de la couronne fonctionnelle annulaire 42. Toutefois, il est entendu que la couronne fonctionnelle annulaire pourrait également, selon d'autre modes de réalisation, être formée à partir d'une pluralité de pièces mécaniques distinctes, assemblées les unes autres d'un seul tenant de sorte que la rotation l'une de ces pièces mécaniques autour de l'axe géométrique de rotation R entre la rotation de l'ensemble d'entre elles.

Il convient de signaler que, par convention, les objets dits « stationnaires » font parties du dispositif de jonction étanche 40 et sont donc destinés à être liés et fixés à demeure sur l'enceinte 10. À l'inverse, les objets dits « embarqués » sont destinés à être supportés par les conteneurs 20 et sont donc mobiles avec ces derniers.

La couronne fonctionnelle annulaire 42, plus particulièrement illustré par les figures 3a et 3b, présente une portion stationnaire d'entrainement en rotation 48 agencée à proximité du pourtour périphérique externe de la couronne fonctionnelle annulaire 42 et formée par des dents 48a orientées radialement et susceptibles d'être entraînées par un moteur d'entraînement 48b placés à l'extérieur de l'enceinte 10 ou bien, selon une réalisation alternative, par un levier (non représenté) activé manuellement. Sous l'impulsion de ce moteur d'entraînement 48b, la couronne fonctionnelle annulaire est donc capable d'entrée en rotation autour de l'axe géométrique de rotation R, dans un sens unidirectionnel. Par convention, il sera considéré par la suite que le sens unidirectionnel est le sens horaire. Toutefois, de manière alternative, le sens de rotation de la couronne fonctionnelle annulaire pourrait aussi bien correspondre au sens antihoraire.

Le dispositif de jonction étanche 40 comporte des moyens stationnaires de bridage temporaire 50.

Les moyens stationnaires de bridage temporaire 50 ont pour fonction de maintenir le conteneur 20 bridé contre l'enceinte 10 de sorte que le couvercle amovible 28 de ce conteneur 20 soit appliqué hermétiquement contre la porte amovible 18 de l'enceinte 10.

Ces moyens stationnaires de bridage temporaire 50 sont liés mécaniquement à la couronne fonctionnelle annulaire 42 de sorte que la rotation de cette couronne fonctionnelle annulaire 42 autour de l'axe géométrique de rotation R dans le sens horaire entraîne mécaniquement l'actionnement des moyens stationnaires de bridage temporaire 50.

Selon la réalisation de la figure 1, les moyens stationnaires de bridage temporaire 50 comprennent quatre agencements fonctionnels stationnaires de bridage temporaire 52 régulièrement répartis sur la couronne fonctionnelle annulaire 42. Chacun de ces agencements fonctionnels stationnaires de bridage temporaire 52 est formé à partir d'une portion de couronne fonctionnelle formant bridage 54 appartenant à la couronne fonctionnelle annulaire 42. Chaque portion de couronne fonctionnelle formant bridage 54 présente ainsi, d'une part, une surface fonctionnelle de bridage axial 56 et, d'autre part, une ouverture d'introduction 58 de moyens embarqués de bridage complémentaire agencés sur une portion de la périphérie extérieure du conteneur (décrits ultérieurement).

Dans la réalisation de la figure 1, la surface fonctionnelle de bridage axial 56 est réalisée sur le pourtour périphérique interne 44_{INT} de la couronne fonctionnelle annulaire 42 par un prolongement de matière décalé axialement de la face extérieure de la paroi périphérique 12 de l'enceinte 10 et interrompu régulièrement pour former l'ouverture d'introduction 58. Toutefois, des réalisations alternatives assurant le bridage axial de la bride annulaire 30 du conteneur 20 contre la face extérieure de la paroi périphérique 12 de l'enceinte 10 pourraient également être envisagées.

Le dispositif de jonction étanche 40 comporte des moyens stationnaires de déverrouillage 60.

Les moyens stationnaires de déverrouillage 60 sont destinés à faire passer le conteneur 20 d'une position de verrouillage initial dans laquelle le couvercle amovible 28 obture hermétiquement le conteneur 20 à une position de déverrouillage intermédiaire dans laquelle le couvercle amovible 28 est désolidarisé du conteneur et maintenu hermétiquement contre la porte 18 de l'enceinte 10 de manière à assurer une communication aseptique entre le conteneur 20 et l'enceinte 10.

Comme précédemment, ces moyens stationnaires de de déverrouillage 60 sont liés mécaniquement à la couronne fonctionnelle annulaire 42 de sorte que la rotation de cette couronne fonctionnelle annulaire 42 autour de l'axe géométrique de rotation R et dans le sens horaire entraîne mécaniquement l'actionnement des moyens de déverrouillage 60. Toutefois, compte-tenu de la disposition de ces moyens stationnaires de déverrouillage 60 par rapport aux moyens stationnaires de bridage temporaire 50, l'actionnement des moyens stationnaires de déverrouillage 60 n'intervient qu'à l'issue du bridage axial du conteneur 20 contre l'enceinte 10.

Selon la réalisation de la figure 1, également visible sur les figures 3a et 3b, les moyens stationnaires de déverrouillage 60 comportent plusieurs agencements fonctionnels stationnaires de déverrouillage 62 - en l'occurrence quatre agencements - régulièrement répartis autour de la couronne fonctionnelle annulaire 42.

Chacun de ces agencements fonctionnels stationnaires de déverrouillage 62 comprend, en premier lieu, une portion de couronne fonctionnelle formant came radiale 64 à profilé interne et/ou externe qui appartient à la couronne fonctionnelle annulaire 42. En l'espèce, la portion de couronne fonctionnelle formant came radiale 64 est réalisée à partir d'un chemin de guidage 66 réalisé dans la couronne fonctionnelle annulaire 42 mais cette réalisation n'est pas limitative.

Chacun de ces agencements fonctionnels stationnaires de déverrouillage 62 comprend, en second lieu, un élément poussoir 68 à déplacement radial capable de coopérer avec la portion de couronne fonctionnelle formant came radiale 64. De cette façon, lors de la rotation de la couronne fonctionnelle 42 jusqu'à une position de déverrouillage intermédiaire, le mouvement relatif de la couronne fonctionnelle annulaire 42 vis-à-vis de l'élément poussoir 68 - qui reste fixe en rotation car il est bloqué dans un aménagement complémentaire 68' - provoque le déplacement dudit élément poussoir 68 dans la direction radiale et fait passer le conteneur 20 d'une position de verrouillage initial à une position de déverrouillage intermédiaire (décrites ultérieurement).

Plus particulièrement, selon le mode de réalisation exemplatif et non limitatif de la figure 1, l'élément poussoir 68 qui est fixe en rotation par rapport à la paroi périphérique 12 de l'enceinte 10 comporte, notamment, un galet 68a agencé dans le chemin de guidage 66 et un téton d'activation 68b relié au galet 68a pour que le déplacement de ce dernier dans la direction radiale entraîne un mouvement radial du téton d'activation 68b, jusqu'au passage du conteneur 20 en position de déverrouillage intermédiaire.

Il convient de noter que les termes « déplacement dans la direction radiale » évoqués précédemment doivent être entendus largement et peuvent ainsi correspondre à une direction passant par l'axe géométrique de rotation R ou bien à une direction légèrement inclinée et formant un angle α avec par rapport à la direction radiale de la bride annulaire 30.

Le dispositif de jonction étanche 40 comporte des moyens stationnaires de verrouillage 70.

Les moyens stationnaires de verrouillage 70 sont destinés à faire passer le conteneur 20 de la position de déverrouillage intermédiaire à une position de verrouillage final dans laquelle ledit couvercle amovible 28 obture, à nouveau, le conteneur 20 de façon hermétique.

Comme précédemment, ces moyens stationnaires de verrouillage 70 sont liés mécaniquement à la couronne fonctionnelle annulaire 42 de sorte que la rotation de cette couronne fonctionnelle annulaire 42 autour de l'axe géométrique de rotation R et dans le sens horaire entraîne mécaniquement l'actionnement des moyens stationnaires de verrouillage 70. De la même façon que précédemment, la position relative des moyens stationnaires de verrouillage 70 et des moyens stationnaires de déverrouillage 60 autour de la couronne fonctionnelle annulaire 42 est telle que l'actionnement de ces moyens stationnaires de verrouillage 70 ne peut être que consécutif à l'arrêt des moyens stationnaires de déverrouillage 60.

Selon la réalisation de la figure 1, visible sur les figures 3a et 3b, les moyens stationnaires de verrouillage 70 comportent quatre agencements fonctionnels stationnaires de déverrouillage 72 régulièrement répartis autour de la couronne fonctionnelle annulaire 42.

Chacun de ces agencements fonctionnels stationnaires de déverrouillage 72 comprend, d'abord, une portion de couronne fonctionnelle formant came radiale 74 à profilé interne et/ou externe qui appartient à la couronne fonctionnelle annulaire 42. Comme précédemment, cette portion de couronne fonctionnelle formant came radiale 74 est réalisée à partir d'un chemin de guidage 76 réalisé dans la couronne fonctionnelle annulaire 42 mais cette réalisation n'est pas limitative.

Chacun de ces agencements fonctionnels stationnaires de déverrouillage 72 comprend, ensuite, un élément poussoir 78 à déplacement radial capable de coopérer avec la portion de couronne fonctionnelle formant came radiale 74. De cette façon, lors de la rotation de la couronne fonctionnelle 42 jusqu'à une position de verrouillage final, le mouvement relatif de la couronne fonctionnelle annulaire 42 vis-à-vis de l'élément poussoir 78 - qui reste fixe en rotation car il est bloqué dans un aménagement complémentaire 68' - provoque le déplacement dudit élément poussoir 78 dans la direction radiale et fait passer le conteneur 20 de la position de déverrouillage intermédiaire à une position de verrouillage final (décrite ultérieurement).

Plus particulièrement, selon le mode de réalisation exemplatif et non limitatif de la figure 1, l'élément poussoir 78 qui est fixe en rotation par rapport à la paroi périphérique 12 de l'enceinte 10 est le même que l'élément poussoir 68 appartenant aux moyens fonctionnels stationnaires de déverrouillage 60. Cet élément poussoir 78 comporte ainsi un galet 68a agencé dans le chemin de guidage 76 et un téton d'activation 68b relié au galet 68a pour que le déplacement de ce dernier dans la direction radiale entraîne un mouvement radial du téton d'activation 68b, jusqu'au passage du conteneur 20 en position de déverrouillage intermédiaire.

Il en découle que la portion de couronne fonctionnelle formant came radiale 74 de l'agencement fonctionnel stationnaire de verrouillage 72 est agencée dans le prolongement de la portion de couronne fonctionnelle formant came radiale 64 l'agencement fonctionnel stationnaire de déverrouillage 62 eu égard au sens de rotation unidirectionnelle de la couronne fonctionnelle annulaire 42. À cet égard, les portions de couronne formant came radiale 72, 62 de l'agencement fonctionnel stationnaire de verrouillage 74 et de l'agencement fonctionnel stationnaire de déverrouillage 62 sont formées par un chemin de guidage 66, 76 continue agencé dans la couronne fonctionnelle annulaire 42 et l'élément poussoir 68, 78 à déplacement radial comporte un galet 68a agencé dans le chemin de guidage 66, 76 continue de sorte que la rotation de la couronne fonctionnelle annulaire génère un déplacement radial du galet 68a qui entraîne le déplacement correspondant d'un téton d'activation 38b.

À l'inverse, selon une alternative (non représentée), les moyens stationnaires de déverrouillage 60 et les moyens stationnaires de verrouillage 70 pourraient éventuellement être structurellement et fonctionnellement distincts et indépendants les uns des autres.

Le dispositif de jonction étanche 40 comporte des moyens stationnaires de blocage/déblocage 80.

Ces moyens stationnaires de blocage/déblocage 80 sont destinés à empêcher/autoriser l'ouverture de la porte amovible 18 de l'enceinte 10 et sont reliés mécaniquement à la couronne fonctionnelle annulaire 42 de sorte que la rotation unidirectionnelle de cette couronne fonctionnelle annulaire 42 autour de l'axe géométrique de rotation R et dans le sens horaire entraîne mécaniquement l'actionnement des moyens stationnaire de blocage/déblocage 80. Plus particulièrement, du fait de la position de ces moyens stationnaire de blocage/déblocage 80 sur la couronne fonctionnelle annulaire 42, ces derniers assurent le déblocage de la porte amovible 18 de l'enceinte 10. Selon la réalisation de la figure 1, les moyens stationnaires de blocage/déblocage 80 assurent le déblocage de la porte amovible 18 après que l'actionnement des moyens stationnaires de déverrouillage 60 ait conduit à faire passer le conteneur 20 en position de déverrouillage intermédiaire. De cette façon, l'ouverture de la porte amovible 18 de l'enceinte 10 est retardée *sine die* lorsqu'un problème se produit au cours du déverrouillage intermédiaire du conteneur 20. Cela permet de limiter les problématiques de confinement - nécessairement plus complexe à réparer au sein de l'enceinte 10 - qui pourraient résulter d'une erreur de manipulation du conteneur 20. De façon similaire, ces moyens stationnaires de blocage/déblocage 80 sont agencés sur la couronne fonctionnelle annulaire 42 afin d'assurer le blocage de la porte amovible 18 de l'enceinte 10 avant que les moyens stationnaires de verrouillage 70 aient été activés pour faire passer le conteneur 20 dans la position de verrouillage final.

Selon un autre mode de réalisation, il serait toutefois aussi possible d'agencer les moyens stationnaires de blocage/déblocage 80 sur la couronne fonctionnelle annulaire 42 de sorte que ces derniers assurent le déblocage de la porte amovible 18 simultanément au passage du conteneur 20 en position de déverrouillage intermédiaire, puis le blocage de cette porte amovible 18 simultanément au passage du conteneur 20 en position de verrouillage final.

Enfin, selon un troisième mode de réalisation, il serait également envisageable d'agencer les moyens stationnaires de blocage/déblocage 80 sur la couronne fonctionnelle annulaire 42 de sorte que ces derniers assurent, d'une part, le déblocage de la porte amovible 18 de l'enceinte 10 avant le passage du conteneur 20 en position de déverrouillage intermédiaire et, d'autre part, le blocage de la porte amovible 18 de l'enceinte 10 après le passage du conteneur 20 en position de verrouillage final.

Les moyens stationnaires de blocage/déblocage 80 tel qu'illustrés par la figure 1 comprennent au moins un agencement fonctionnel stationnaire de blocage/déblocage 82 formé par une portion de couronne fonctionnelle formant engrenage 84 appartenant à la couronne fonctionnelle annulaire 42. À ce titre, cette portion de couronne fonctionnelle formant engrenage 84 est positionnée à l'extérieur de l'enceinte 10.

Par ailleurs, selon la réalisation de la figure 1, cette portion de couronne fonctionnelle formant engrenage 84 est crantée de manière à engrainer un arbre de rotation 86, traversant la paroi périphérique 12 de l'enceinte 10 et entraînant lui-même un organe de blocage 88 lors de la rotation unidirectionnelle de la couronne fonctionnelle. Plus particulièrement, l'organe de blocage 88 - qui est se trouve dans l'espace intérieur 14 de l'enceinte 10 - est agencé de manière à pouvoir passer d'une position bloquée dans laquelle une portion à recouvrement 88a empêche la porte amovible 18 de l'enceinte 10 de s'ouvrir à une position débloquée dans laquelle la portion à recouvrement 88a n'empêche plus la porte amovible 18 de s'ouvrir.

Le dispositif de jonction étanche 40 comporte aussi des moyens stationnaires de manoeuvre 90.

Ces moyens stationnaires de manoeuvre 90 sont destinés à ouvrir et refermer hermétiquement la porte amovible 18 de l'enceinte 10.

Pour ce faire, les moyens stationnaires de manoeuvre 90 peuvent, selon un premier mode de réalisation (non représenté), être entraînés mécaniquement par la couronne fonctionnelle annulaire 42. Dans ce cas de figure, les moyens stationnaire de manoeuvre 90 sont reliés mécaniquement à la couronne fonctionnelle annulaire 42 de sorte que la rotation unidirectionnelle de cette couronne fonctionnelle annulaire 42 autour de l'axe géométrique de rotation R et dans le sens horaire entraîne mécaniquement l'actionnement des moyens stationnaires de manoeuvre 90. Plus particulièrement, la position de ces moyens stationnaires de manoeuvre 90 sur la couronne fonctionnelle annulaire 42 permet d'ouvrir la porte amovible 18 de l'enceinte 10 après le passage du conteneur 20 en position de déverrouillage, puis de refermer la porte amovible 18 avant le passage du conteneur 20 en position de verrouillage final.

Selon un second mode de réalisation illustré par la figure 1, les moyens stationnaires de manoeuvre 90 sont entraînés par un moteur de manoeuvre 92 asservi sur le déplacement de la couronne fonctionnelle annulaire 42.

Ces moyens stationnaires de manoeuvre 90 comportent alors un bras rotatif 94 apte à se déplacer en rotation autour d'un axe de rotation horizontal ainsi qu'un bras translatif 96 apte à se déplacer le long d'un axe horizontal. De cette façon, les moyens stationnaires de manoeuvre 90 permettent, grâce au moteur de manoeuvre 92 de déplacer la porte amovible 18 de l'enceinte 10, d'abord, dans une direction axiale, puis ensuite, dans une direction sensiblement perpendiculaire à la direction axiale de sorte que la porte amovible 18 n'entrave pas le passage du produit biopharmaceutique lors de leur transfert aseptique entre l'enceinte 10 et le conteneur 20.

Il convient de noter que, selon l'invention, les moyens stationnaires de bridage temporaire 50, les moyens stationnaires de déverrouillage 60, les moyens stationnaires de verrouillage 70 et éventuellement les moyens de blocage/déblocage 80 et les moyens de manoeuvre 90 sont liés mécaniquement à la couronne fonctionnelle annulaire 42 et agencés de sorte que la rotation unidirectionnelle de ladite couronne fonctionnelle annulaire 42 autour de l'axe géométrique de rotation R et dans le sens horaire entraîne successivement l'actionnement des moyens stationnaires de bridage temporaire 50 pour assurer le maintien en position du conteneur 20 contre l'enceinte 10, puis simultanément ou successivement l'actionnement des moyens stationnaires de déverrouillage 60 assurant le passage en position de déverrouillage intermédiaire du conteneur 20 et l'actionnement des moyens stationnaires de blocage/déblocage 80 assurant le déblocage de la porte amovible 18 de l'enceinte 10, puis simultanément ou successivement l'actionnement des moyens stationnaires de blocage/déblocage 80 assurant le blocage de la porte amovible 18 de l'enceinte 10 et l'actionnement des moyens stationnaires de verrouillage 70 assurant le passage en position de verrouillage final du conteneur 20, et à nouveau l'actionnement des moyens stationnaires de bridage temporaire 50 du conteneur afin de libérer le conteneur 20.

Au surplus, les moyens stationnaires de bridage temporaire 40 sont agencés de manière à assurer le positionnement du conteneur 20 et une indexation par rapport aux autres moyens fonctionnels. De la même façon, l'accumulation de plusieurs agencements fonctionnels cumulés sur la couronne fonctionnelle annulaire 42 permet la mise en oeuvre d'un cycle de manipulation du conteneur 20 en ne réalisant que 1/n tour de rotation. En l'espèce, le mode de réalisation comporte 4 agencements fonctionnels distincts régulièrement répartis sur la couronne fonctionnelle annulaire mais il serait possible d'en inclure moins - à savoir un, deux ou trois - ou bien d'en inclure davantage.

Il convient de signaler que la rotation unidirectionnelle de la couronne fonctionnelle annulaire 42 permet d'assurer l'actionnement des moyens stationnaires de bridage temporaire, de déverrouillage, de verrouillage, de blocage/déblocage et de manoeuvre que cette couronne fonctionnelle annulaire se déplace dans un sens de rotation ou bien dans l'autre.

Il serait, en outre, aussi possible d'intégrer cette couronne fonctionnelle annulaire 42 non pas à l'extérieur de l'enceinte 10 comme illustré par la réalisation de la figure 1 mais directement dans l'espace intérieur 14 de l'enceinte 10, contre la face intérieure de la paroi périphérique 12. Cette réalisation permettrait, notamment, d'éviter l'utilisation de moyens de blocage/déblocage 80 présentant un arbre de rotation 86 traversant la paroi périphérique 12.

La figure 4 représente de manière plus détaillée un mode de réalisation d'un conteneur destiné à être associé au dispositif de jonction étanche selon l'invention.

Toutefois, dans cette illustration, l'enveloppe périphérique du conteneur 20 n'est pas représentée.

Comme indiqué précédemment, ce conteneur 20 est destiné à permettre le transport et le transfert aseptique de produits biopharmaceutiques depuis ou vers une enceinte 10 dotée du dispositif de jonction étanche 40 selon l'invention.

À cet égard, le conteneur 20 comprend la bride annulaire 30 délimitant l'ouverture annulaire 26, le couvercle amovible 28 apte à obturer hermétiquement l'ouverture 26 de la bride annulaire 30 et l'enveloppe périphérique solidaire de la bride annulaire 30 et délimitant l'espace intérieur 24 confiné susceptible de contenir les produits biopharmaceutiques. De cette façon, lorsqu'il est fermé par le couvercle amovible 28 le conteneur 20 est hermétiquement clos, ce qui prévient toute fuite ou toute introduction de matière dans l'espace intérieur 24. Le conteneur 20 peut être rigide ou souple et réutilisable ou à usage unique selon les cas de figure. À titre exemplatif et nullement limitatif, il peut s'agir d'un conteneur 20 de toutes tailles, tel qu'une poche, une gaine, un récipient, un bioréacteur, une goulotte, etc.

Le conteneur 20 comprend également des moyens embarqués de bridage temporaire 100.

Ces moyens embarqués de bridage temporaire 100 sont agencés sur un pourtour périphérie externe de la bride annulaire 30 de manière à permettre le bridage temporaire du conteneur 20 par blocage axial sur la face extérieure de la paroi périphérique 10 de l'enceinte 10 via l'actionnement du dispositif de jonction étanche 40.

Plus particulièrement, selon la réalisation de la figure 4, les moyens embarqués de bridage temporaire 100 comprennent au moins un agencement fonctionnel embarqué de bridage temporaire 102 formé par au moins un ergot 104, de préférence deux ergots 104, présentant une surface fonctionnelle embarquée de bridage axial 106 apte à venir en appui contre la surface fonctionnelle stationnaire de bridage axial 56 du dispositif de jonction étanche 40. Ces ergots 104 susceptibles d'être positionnés entre la face extérieure de la paroi périphérique 12 de l'enceinte 10 et la surface fonctionnelle stationnaire de bridage axial 56 du dispositif de jonction étanche 40 en passant, d'abord, au travers de l'une des ouvertures d'introduction 58 de ce dispositif de jonction étanche 40 permet de bloquer axialement le déplacement du conteneur 20 vis-à-vis de l'enceinte 10.

Lorsque le conteneur 20 est placé bridé contre l'enceinte 10, le couvercle amovible 28 du conteneur 20 est maintenu à demeure - par aimantation ou autre - de manière hermétique contre la porte amovible 108 de l'enceinte 10. De cette façon, l'espace extérieur confiné entre le couvercle amovible 28 et la porte amovible 18 ne peut s'échapper lors du transfert aseptique.

Le conteneur 20 comprend également des moyens embarqués de verrouillage/déverrouillage 110 du couvercle amovible 28 sur la bride annulaire 30.

Ces moyens embarqués de verrouillage/déverrouillage 110 - dont la structure est détaillée ci-après - ont pour fonction d'assurer le maintien du conteneur 20 dans trois positions distinctes. La première position est qualifiée de position de verrouillage initiale car les moyens de verrouillage/déverrouillage 110 interdisent le déplacement relatif du couvercle amovible 28 vis-à-vis de la bride annulaire 30. La deuxième position, qualifiée de position de déverrouillage intermédiaire, est censée intervenir lorsque la bride annulaire 30 du conteneur 20 est bridé axialement contre la paroi périphérique 12 de l'enceinte 10 et autorise le déplacement le relatif du couvercle amovible 28 vis-à-vis de la bride annulaire 30. La troisième position, qualifiée de position de verrouillage final, est supposée intervenir après le transfert aseptique des produits biopharmaceutiques entre le conteneur 20 et l'enceinte 10 et interdit, à nouveau et de manière réversible ou irréversible, le déplacement relatif du couvercle amovible 28 vis-à-vis de la bride annulaire 30.

Pour assurer ce passage de la position de verrouillage initiale à la position de déverrouillage intermédiaire, puis à la position de verrouillage final, les moyens embarqués de verrouillage/déverrouillage 110 selon la réalisation de la figure 4 comportent quatre agencements fonctionnels embarqués de verrouillage/déverrouillage 112 régulièrement répartis autour de la bride annulaire 28 du couvercle 30.

Chaque agencement fonctionnel embarqué de verrouillage/déverrouillage 112 comporte un logement traversant 114 formé dans la bride annulaire 30 ainsi qu'un logement borgne 116 formé dans le couvercle amovible 28 du conteneur 20 et dans le prolongement du logement traversant 114.

Chaque agencement fonctionnel embarqué de verrouillage/déverrouillage 112 comporte également une goupille à positionnement radial interne 118 et une goupille à positionnement radial externe 120. Ces deux goupilles à positionnement radial interne 118 et externe 120 présentent, selon la réalisation de la figure 4, un corps allongé de forme cylindrique leur permettant d'être introduites et de se déplacer à l'intérieur du logement borgne 116 formé dans le couvercle amovible 28, d'une part, et du logement traversant 114 agencé dans la bride annulaire 30, d'autre part. Il convient toutefois de noter que les caractéristiques dimensionnelles et les propriétés de la surface extérieure de ces goupilles à positionnement radial interne 118 et externe 120 sont tels que ces dernières ne peuvent se déplacer à l'intérieur des logements borgne 116 et traversant 114 du seul fait de la gravité. En outre, ces goupilles à positionnement radial interne 118 et externe 120 pourraient présenter un corps de forme différente - par exemple, parallélépipédique - afin de parvenir au même résultat.

Selon la réalisation de la figure 4, logement traversant 114, le logement borgne 116, la goupille à positionnement radial interne 118 et la goupille à positionnement radial externe 120 sont coaxiaux et orientés dans une direction radiale relative à la bride annulaire 30. Plus exactement, selon cette réalisation, ces différents éléments s'étendent selon une direction sensiblement radiale, orientée vers l'axe géométrique de révolution R. Toutefois, selon une réalisation alternative, lesdits logements traversant 114, logement borgne 116, goupille à positionnement radial interne 118 et goupille à positionnement radial externe 120 pourraient également être orientés dans une direction légèrement inclinée et formant un angle α par rapport par rapport à une direction radiale relative à la bride annulaire 30.

D'autre part, selon la réalisation de la figure 4, le logement borgne 116 affleure le logement traversant 114 mais il pourrait également être envisagé de formé un espace intermédiaire entre le logement borgne 116 et le logement traversant 114 sans que cela nuise au confinement et à la manipulation du co nteneur 20.

Lorsque le conteneur 20 se trouve dans la position de verrouillage initiale, comme illustré par la figures 5a, la goupille à positionnement radial interne 118 présente une portion fonctionnelle interne de verrouillage initial 118_{INT} agencée dans le logement borgne 116 du couvercle amovible 28 et une po rtion fonctionnelle externe de verrouillage initial 118_{EXT} agencée dans le logement traversant 114 de la bride annulaire 30. Ainsi, la goupille à positionnement radial interne 118 interdit le déplacement relatif du couvercle amovible 28 vis-à-vis de la bride annulaire 30.

Par ailleurs, dans cette même position de verrouillage initiale, la goupille à positionnement radial externe 120 est au moins partiellement agencée dans le logement traversant 114 de la bride annulaire 30. Selon un premier mode de réalisation (représenté sur la figure 5a), la goupille à positionnement radial externe 120 peut alors présenter une partie extrême fonctionnelle à positionnement radial externe 122 exclue du logement traversant 114. L'accès à cette partie extrême fonctionnelle 122 par les moyens stationnaires de déverrouillage 60 ou de verrouillage 70 est ainsi facilité. Selon un deuxième mode de réalisation (non représenté), cette goupille à positionnement radial externe 120 peut alternativement présenter une partie extrême fonctionnelle à positionnement radial externe 122 affleurant l'extrémité externe débouchant du logement traversant 114. Ainsi, l'accès à cette partie extrême fonctionnelle 122 par les moyens stationnaires de déverrouillage 60 ou de verrouillage 70 reste relativement aisé mais les risques de manipulation inopinée de la goupille à positionnement externe 120 sont néanmoins réduits. Enfin, selon un troisième mode de réalisation (non représenté), ladite goupille à positionnement radial externe 120 peut présenter une partie extrême fonctionnelle à positionnement radial externe 122 entièrement logée à l'intérieur du logement traversant 114. De cette façon, la manipulation de la goupille à positionnement externe 120 est quasiment impossible sans l'utilisation d'un outil spécifique.

Lorsque le conteneur 20 se trouve dans la position de déverrouillage intermédiaire, comme illustré par la figure 5b, la goupille à positionnement radial interne 118 est au moins partiellement agencée dans le logement borgne 116 du couvercle amovible 28 et entièrement exclue du logement traversant 114 de la bride annulaire 30. De cette façon, cette goupille à positionnement radial interne 118 n'interdit plus les déplacement relatif de la bride annulaire 30 et du couvercle amovible 28 du conteneur 20.

Dans cette position de déverrouillage intermédiaire, toujours, la goupille à positionnement radial externe 120 est au moins partiellement agencée dans le logement traversant 114 de la bride annulaire 30 et entièrement exclue du logement borgne 116 du couvercle amovible 28. De cette façon, la goupille à positionnement radial externe 120 n'a pas plus d'influence que la goupille à positionnement radial interne 118 sur le déplacement relatif du couvercle amovible 28 vis-à-vis de la bride annulaire 30 qui peuvent donc librement être désolidarisé pour autoriser le transfert aseptique des produits biopharmaceutiques.

De la même façon que précédemment, la partie extrême fonctionnelle à positionnement radial externe 122 de ladite goupille à positionnement radial externe 120 peut alors soit être exclue du logement traversant 114, soit affleurer l'extrémité externe débouchant du logement traversant 114, soit être logée entièrement à l'intérieur dudit logement traversant 114.

Enfin, pour placer le conteneur 20 dans la position de verrouillage final, comme illustré par la figure 5c, il convient en premier lieu de replacer le couvercle amovible 28 contre la bride annulaire 30 puis de placer l'agencement fonctionnel embarqué de verrouillage/déverrouillage 112 dans la position appropriée.

Plus particulièrement, dans cette position la goupille à positionnement radial interne 118 est entièrement agencée à l'intérieur du logement borgne 116 du couvercle amovible 28 tandis que la goupille à positionnement radial externe 120 présente une portion fonctionnelle interne de verrouillage final 120_{INT} agencée dans le logement borgne 116 du couvercle amovible 28 et une portion fonctionnelle externe de verrouillage final 120_{EXT} agencée dans le logement traversant 114 de la bride annulaire 30. Par conséquent, le logement borgne 116 du couvercle amovible 28 présente une longueur suffisante pour accueillir, d'une part, la goupille à positionnement radial interne 118 et d'autre part, la portion fonctionnelle interne de verrouillage final 120_{INT}. Ainsi, la goupille à positionnement radial externe 120 interdit le déplacement relatif du couvercle amovible 28 vis-à-vis de la bride annulaire 30.

À nouveau, la partie extrême fonctionnelle à positionnement radial externe 122 de ladite goupille à positionnement radial externe 120 peut alors être exclue du logement traversant 114 afin de faciliter l'éventuelle réouverture ultérieure du conteneur 20. Inversement, ladite partie extrême fonctionnelle à positionnement radial externe 122 peut affleurer l'extrémité externe débouchant du logement traversant 114 ou bien être logée entièrement à l'intérieur dudit logement traversant 114 afin de limiter les risque d'ouverture ultérieure du conteneur 20.

Une telle utilisation des goupilles à positionnement radial interne 118 et externe 120 pour placer le conteneur en position de verrouillage initial, de déverrouillage intermédiaire et de verrouillage final permet de n'avoir qu'un logement borgne 116 et un logement traversant 114 à réaliser dans le couvercle amovible 28 et la bride annulaire 30 par agencement fonctionnel embarqué de verrouillage/déverrouillage 112, ce qui limite les risques de contamination lié aux éventuels défauts de production. En outre, la manipulation des moyens de verrouillage/déverrouillage 110 est facilitée dans la mesure où il suffit de déplacer les goupilles à positionnement radial interne 118 et externe 120 dans une seule et unique direction et un seul et même sens pour faire passer le conteneur 20 successivement de la position de verrouillage initial à la position de déverrouillage intermédiaire, puis à la position de verrouillage final. La réalisation et le fonctionnement du dispositif de jonction étanche en sont ainsi simplifiés.

Il convient également de noter que dans la réalisation de la figure 4, les goupilles à positionnement radial interne 118 et externe 120 présentent des longueurs identiques, ce qui permet notamment de simplifier la production et l'assemblage des goupilles à positionnement radial interne 118 et externe 120 sur le conteneur puisqu'il n'est alors pas nécessaire de distinguer les goupilles eu égard à leur dimensions respectives. Toutefois, de manière alternative, ces goupilles à positionnement radial interne 118 et externe 120 pourraient également présenter des longueurs différentes.

Il convient en outre de souligner que le logement traversant 114 et la goupille à positionnement radial externe 120 présentent des longueurs telles que, lorsque les moyens embarqués de verrouillage/déverrouillage 110 sont dans la position de déverrouillage provisoire, la goupille à positionnement radial externe 120 présente une partie extrême fonctionnelle 122 à positionnement radial externe exclue du logement traversant 114. Ce positionnement facilite, notamment, l'opération effectuée par les moyens stationnaires de verrouillage 70 pour faire passer le conteneur 20 de la position de déverrouillage intermédiaire à la position de verrouillage final puisque la goupille à positionnement radial externe 120 est accessible sans que l'élément poussoir à déplacement radial 78 n'ait à se déplacer jusqu'à l'intérieur du logement traversant 114.

En revanche, les longueurs de la goupille à positionnement radial externe 120 et du logement traversant 114 sont telles que, lorsque les moyens embarqués de verrouillage/déverrouillage 110 sont dans la position de verrouillage final, ladite goupille à positionnement radial externe 120 présente une partie extrême fonctionnelle 120 à positionnement radial externe logée à l'intérieure du logement traversant. De cette façon, lorsque le conteneur est placé en position de verrouillage final, la préhension de la goupille à positionnement radial externe 120 est rendu complexe et irréalisable sans un outil de préhension adapté. Cela permet ainsi d'élever le niveau de sécurité se rapportant à l'étanchéité du conteneur 20 en position de verrouillage final.

À cet égard, il pourrait également être envisagé d'intégrer sur la goupille à position radial externe 120 et sur le logement traversant 114 des moyens complémentaires de protection et d'alerte - formés par exemple par des épaulements complémentaires ou tout élément analogue - empêchant le retrait de la goupille à positionnement radial externe 120 hors du logement traversant 114 après la mise en position du conteneur 20 dans la position de verrouillage final et engendrant la destruction d'une partie de la bride annulaire 30 dans l'éventualité d'un tel retrait.

De manière alternative (non représentée), il serait également possible que les longueurs de la goupille à positionnement radial externe 120 et du logement traversant 114 soient telles que, lorsque les moyens embarqués de verrouillage/déverrouillage 110 sont dans la position de verrouillage final, ladite goupille à positionnement radial externe 120 présente une partie extrême fonctionnelle à positionnement radial externe exclue du logement traversant.

Une telle réalisation permettrait, au contraire, de faciliter le retrait de la goupille à positionnement radial externe 120 en dehors du logement traversant 114 et d'obtenir un conteneur réutilisable après sa mise en position de verrouillage final.

Selon la réalisation de la figure 4, le conteneur 20 comporte également des moyens embarqués de cloisonnement et de protection 124.

Les moyens embarqués de cloisonnement et de protection 124 sont agencés sur une portion périphérique externe de la bride annulaire 30 de manière empêcher la manipulation inopinée des moyens embarqués de verrouillage/déverrouillage portés par la bride annulaire 30.

Selon une réalisation, les moyens embarqués de cloisonnement et de protection 124 comprennent quatre agencements fonctionnels embarqués de cloisonnement et de protection 126 disposés autour des quatre logements traversant 114 de manière à faire obstacle à la manipulation inopinée ou volontaire de la goupille à positionnement radial externe 120 lorsque cette dernière n'est pas entièrement insérée dans le logement traversant 118 de la bride annulaire 30.

Chaque agencement fonctionnel embarqué de cloisonnement et de protection 126 est réalisé à partir de deux ergots 128, disposés autour ou de part et d'autre du logement traversant 114. Selon cette réalisation, les ergots 128 présente des dimensions radiales telles que, en position de verrouillage provisoire, la goupille à positionnement radial externe 120 ne s'étend pas au-delà des ergots 128 appartenant moyens de cloisonnement et de protection.

Toutefois, selon une réalisation alternative ou complémentaire, l'agencement fonctionnel embarqué de cloisonnement et de protection 62 pourrait également être réalisé à partir d'un alésage formé dans la bride annulaire 30, orienté radialement et présentant un diamètre supérieur au diamètre du logement traversant 114. Une telle réalisation permet d'empêcher d'accéder à la goupille à positionnement radial externe 120 tout en conservant de l'espace autour de cette goupille à positionnement radial externe 120 afin de ne pas compliquer l'opération de déplacement de cette dernière par les moyens stationnaires de déverrouillage 60 et de verrouillage 70.

Il convient de noter que, selon la réalisation de la figure 4, les ergots 104 formant les moyens embarqués de bridage temporaire 102 et les ergots 128 formant les moyens embarqués de protection et de cloisonnement 124 sont les mêmes. Ainsi, ces ergots 104, 108 présentent, d'une part, une surface fonctionnelle embarquée de bridage axial 56 assurant le bridage du conteneur contre la paroi périphérique 12 de l'enceinte 10 et, d'autre part, une forme assurant la protection et le cloisonnement de la goupille à positionnement radial externe 120 lorsqu'elle n'est que partiellement insérée à l'intérieur du logement traversant 114. Une telle réalisation permet de limiter les coûts de production et de simplifier la bride annulaire 30 en limitant le nombre d'ergots à réaliser sur le pourtour périphérique externe de cette bride annulaire 30.

Cependant, il serait également envisageable d'utiliser des moyens embarqués de bridage temporaire 102 et des moyens embarqués de protection et de cloisonnement 124 structurellement et fonctionnellement indépendants les uns des autres, en utilisant par exemple des ergots 104 pour le bridage axial et d'autres ergots différents pour le cloisonnement et la protection des goupilles à positionnement radial externe 120.

Il convient de signaler comme précédemment que les moyens embarqués de bridage temporaire 100 sont agencés de manière à assurer une indexation de la position du conteneur 20 par rapport aux autres moyens stationnaires appartenant au dispositif de jonction étanche 40.Cette accumulation d'agencements fonctionnels sur la couronne fonctionnelle annulaire 42 permet la mise en oeuvre d'un cycle de manipulation du conteneur 20 en ne réalisant que 1/n tour de rotation avec la couronne fonctionnelle annulaire 42. En l'espèce, le mode de réalisation comporte quatre agencements fonctionnels distincts régulièrement répartis sur la couronne fonctionnelle annulaire 42 mais il serait possible d'en inclure moins - à savoir un, deux ou trois - ou bien d'en inclure davantage.

La mise en oeuvre du procédé de transfert aseptique selon l'invention est maintenant décrite en détail, notamment en référence à la figure 6.

Ce procédé de transfert aseptique consiste, en premier lieu, à disposer d'une enceinte 10 close telle que décrite précédemment et supportant un dispositif de jonction étanche 40 selon l'invention. Ce procédé de transfert aseptique consiste, en deuxième lieu, à disposer d'un conteneur 20 en position de verrouillage initial tel que décrit précédemment.

Le procédé de transfert aseptique implique ensuite de mettre en place le conteneur 20 contre la paroi périphérique 12 de l'enceinte 10 en introduisant les ergots 104 appartenant aux agencement fonctionnels embarqués de bridage temporaire 102 à travers les ouvertures d'introduction 58 appartenant aux moyens stationnaires de bridage temporaire 50.

Ce faisant, la bride annulaire 30 du conteneur 20 vient se positionner contre la paroi périphérique 12 de l'enceinte 10 et le couvercle amovible 28 - au moins partiellement formé de ferrite - est maintenu hermétiquement en position contre la face extérieure de la porte amovible 18 - au moins partiellement aimantée.

Le procédé de transfert aseptique consiste ensuite à générer la rotation de la couronne fonctionnelle annulaire 42 dans le sens horaire afin que les portions de couronne fonctionnelle formant bridage 54 viennent se positionner en regard des ergots 104 formant les moyens embarqués de bridage temporaires et emprisonnent ces ergots 104 entre la paroi périphérique 12 de l'enceinte 10 et les surfaces fonctionnelles de bridage axial 56.

Le conteneur 20 est ainsi maintenu en position par le dispositif de jonction étanche 40 contre la paroi périphérique 12 de l'enceinte 10.

Le procédé de transfert aseptique consiste ensuite à générer la rotation de la couronne fonctionnelle annulaire 42 dans le sens horaire afin d'actionner l'actionnement des moyens stationnaires de déverrouillage 60. Plus particulièrement, la rotation de cette couronne fonctionnelle annulaire 42 dans le sens horaire génère un déplacement de l'élément poussoir à déplacement radial 68 du fait du changement de position radiale du galet 68a dans le chemin de guidage 66. Le téton d'activation 68b s'abaisse alors et vient pousser la goupille à positionnement radial externe 120 et la goupille à positionnement radial interne 118 par la même occasion.

À l'issue de cette opération, la goupille à positionnement radial interne 118 est alors agencée dans le logement borgne 116 du couvercle amovible 28 et entièrement exclue du logement traversant 114 de la bride annulaire 30 et la goupille à positionnement radial externe 120 est agencée dans le logement traversant 114 de la bride annulaire 30 et entièrement exclue du logement borgne 116 du couvercle amovible 28. De ce fait, le conteneur 20 est en position de déverrouillage intermédiaire et le déplacement le relatif du couvercle amovible 28 vis-à-vis de la bride annulaire 30 est possible.

Le procédé de transfert aseptique consiste également, simultanément ou successivement à l'étape qui précède, à générer la rotation de la couronne fonctionnelle annulaire 42 dans le sens horaire afin d'actionner les moyens stationnaires de blocage/déblocage 80 pour libérer la porte amovible 18 de l'enceinte 10.

À cet effet, les portions de couronne fonctionnelle formant engrenage 84 - qui sont entrainées en rotation avec la couronne fonctionnelle annulaire 42 - engrènent l'arbre de rotation 86 et, donc, l'organe de blocage 88 pour que ce dernier ne recouvre plus la face extérieure de la porte amovible 18 de l'enceinte 10. La porte amovible 18 peut alors être manipulée librement.

Le procédé de transfert aseptique consiste alors à entraîner les moyens stationnaires de manoeuvre 90 pour déplacer la porte amovible 18 de l'enceinte 10 et le couvercle amovible du conteneur afin de libérer les ouvertures annulaires 16, 26 de l'enceinte 10 et du conteneur 20.

Pour ce faire, les moyens stationnaires de manoeuvre 90 -qui peuvent être actionnés par la couronne fonctionnelle annulaire 42 ou bien par un moteur de manoeuvre asservis sur la position de cette couronne fonctionnelle annulaire 42 - entraînent en translation horizontale, d'abord, puis en rotation autour d'un axe horizontal, ensuite, la porte amovible 18 de l'enceinte 10.

Le conteneur 20 étant en position de déverrouillage intermédiaire, le couvercle amovible 28 est capable de se déplacer vis-à-vis de la bride annulaire 30 et de suivre les mouvements de la porte amovible 18 de l'enceinte 10 du fait de la force magnétique les reliant l'une à l'autre. Ainsi, l'enceinte 10 et le conteneur 20 sont-ils ouverts l'un sur l'autre tout en étant hermétiquement isolé du milieu extérieur.

Le transfert aseptique des produits biopharmaceutique entre le conteneur 20 et l'enceinte 10 peut alors s'opérer.

Le procédé de transfert aseptique consiste ensuite à entraîner à nouveau les moyens stationnaires de manoeuvre 90 afin de refermer de façon hermétique les ouvertures annulaires 16, 26 de l'enceinte 10 et du conteneur 20.

Comme précédemment, les moyens stationnaires de manoeuvre 90 entraînent alors en rotation autour d'un axe horizontal puis en translation horizontale la porte amovible 18 de l'enceinte 10 ainsi que le couvercle amovible du conteneur 20 pour les remettre dans la même position que précédemment.

L'enceinte 10 est alors à nouveau obturée hermétiquement par la porte amovible 18, tant vis-à-vis de l'extérieur que du conteneur 10. Et, de façon symétrique, le conteneur 20 est obturé hermétiquement par le couvercle amovible, tant vis-à-vis de l'extérieur que de l'enceinte 10.

Le procédé de transfert aseptique consiste alors à générer la rotation de la couronne fonctionnelle annulaire 42 dans le sens horaire afin d'actionner les moyens stationnaires de blocage/déblocage 80 pour bloquer la porte amovible 18 de l'enceinte 10.

À cet effet, les portions de couronne fonctionnelle formant engrenage 84 - qui sont entrainées en rotation avec la couronne fonctionnelle annulaire 42 - engrènent l'arbre de rotation 86 et, donc, l'organe de blocage 88 pour que ce dernier recouvre à nouveau la face extérieure de la porte amovible 18 de l'enceinte 10. La porte amovible 18 ne peut donc plus être manipulée librement.

Le procédé de transfert aseptique consiste, simultanément ou bien successivement à l'étape précédent à générer la rotation de la couronne fonctionnelle annulaire 42 dans le sens horaire afin d'actionner l'actionnement des moyens stationnaires de verrouillage 70. Plus particulièrement, la rotation de cette couronne fonctionnelle annulaire 42 dans le sens horaire génère un déplacement de l'élément poussoir à déplacement radial 78 du fait du changement de position radiale du galet 68a dans le chemin de guidage 76. Le téton d'activation 68b s'abaisse alors et vient pousser la goupille à positionnement radial externe 120 et la goupille à positionnement radial interne 118 par la même occasion.

À l'issue de cette opération, la goupille à positionnement radial interne 118 est agencée dans le logement borgne 116 du couvercle amovible 28 tandis que la goupille à positionnement radial externe 120 présente une portion fonctionnelle interne de verrouillage final 120_{INT} agencée dans le logement borgne 116 du couvercle amovible 28 et une portion fonctionnelle externe de verrouillage final 120_{EXT} agencée dans le logement traversant 114 de la bride annulaire 30. Ainsi le déplacement relatif du couvercle amovible 28 vis-à-vis de la bride annulaire 30 est impossible et le conteneur est alors en position de verrouillage final.

Le procédé de transfert aseptique consiste dès lors à générer la rotation de la couronne fonctionnelle annulaire 42 dans le sens horaire afin que les portions de couronne fonctionnelle formant bridage temporaire 54 se positionnent de sorte que les ouvertures d'introduction 58 soient en regard des ergots 104 formant les moyens embarqués de bridage temporaires et les libèrent.

Le conteneur 20 en position de verrouillage finale peut ainsi être librement retiré du dispositif de jonction étanche 40.

## Revendications

1. **Conteneur** (20) spécialement destiné à assurer le transport et le transfert aseptique d'un produit biopharmaceutique depuis ou vers une enceinte (10) close dotée d'un dispositif de jonction étanche (40), comprenant :
une bride annulaire (30) délimitant une ouverture (26) ;
un couvercle amovible (28) apte à obturer hermétiquement l'ouverture (26) de la bride annulaire (30) ;
des moyens embarqués de verrouillage/déverrouillage (110) du couvercle amovible (28) sur la bride annulaire (30) ; et
une enveloppe périphérique (22) solidaire de la bride annulaire (30) et délimitant un espace intérieur (24) confiné apte à recevoir les produits appartenant au domaine biopharmaceutique ;
dans lequel les moyens embarqués de verrouillage/déverrouillage (110) comprennent au moins un agencement fonctionnel embarqué de verrouillage/déverrouillage (112) formé, d'une part, par un logement traversant (114) formé dans la bride annulaire (30) et un logement borgne (116) formé dans le couvercle amovible (28) et dans le prolongement du logement traversant (114) lorsque le couvercle amovible (28) obture hermétiquement l'ouverture de la bride annulaire (30) et, d'autre part, par une goupille à positionnement radial interne (118) et une goupille à positionnement radial externe (120) susceptibles d'être introduites et de se déplacer à l'intérieur du logement borgne (116) du couvercle amovible (28) et du logement traversant (114) de la bride annulaire (30) ;
le conteneur (20) étant apte à se trouver dans une position de verrouillage initial dans laquelle, d'une part, la goupille à positionnement radial interne (118) présente une portion fonctionnelle interne de verrouillage initial (118_{INT}) agencée dans le logement borgne (116) du couvercle amovible (28) et une portion fonctionnelle externe de verrouillage initial (118_{EXT}) agencée dans le logement traversant (114) de la bride annulaire (30) de manière à interdire le déplacement relatif du couvercle amovible (28) vis-à-vis de la bride annulaire (30) et, d'autre part, la goupille à positionnement radial externe (120) est au moins partiellement agencée dans le logement traversant (114) de la bride annulaire (30) ;
le conteneur (20) étant apte à se trouver dans une position de déverrouillage intermédiaire dans laquelle, d'une part, la goupille à positionnement radial interne (118) est au moins partiellement agencée dans le logement borgne (116) du couvercle amovible (28) et entièrement exclue du logement traversant (114) de la bride annulaire (30) et, d'autre part, la goupille à positionnement radial externe (120) (120) est au moins partiellement agencée dans le logement traversant (114) de la bride annulaire (30) et entièrement exclue du logement borgne (116) du couvercle amovible (28) de sorte à autoriser le déplacement le relatif du couvercle amovible (28) vis-à-vis de la bride annulaire (30) ;
**caractérisé en ce que** le conteneur (20) est apte à se trouver dans une position de verrouillage final dans laquelle, d'une part, la goupille à positionnement radial interne (118) est agencée dans le logement borgne (116) du couvercle amovible (28) et, d'autre part, la goupille à positionnement radial externe (120) présente une portion fonctionnelle interne de verrouillage final (120_{INT}) agencée dans le logement borgne (116) du couvercle amovible (28) et une portion fonctionnelle externe de verrouillage final (120_{EXT}) agencée dans le logement traversant (114) de la bride annulaire (30) de manière à interdire le déplacement relatif du couvercle amovible (28) vis-à-vis de la bride annulaire (30).

2. Conteneur (20) selon la revendication 1, dans lequel le logement traversant (114) et la goupille à positionnement radial externe (120) présentent des longueurs telles que, lorsque les moyens embarqués de verrouillage/déverrouillage (110) sont dans la position de verrouillage initial, ladite goupille à positionnement radial externe (120) présente une partie extrême fonctionnelle à positionnement radial externe (122) soit exclue du logement traversant (114), soit logée à l'intérieur du logement traversant (114), soit affleurant l'extrémité externe débouchant du logement traversant (114).

3. Conteneur (20) selon la revendication 1, dans lequel le logement traversant (114) et la goupille à positionnement radial externe (120) présentent des longueurs telles que, lorsque les moyens embarqués de verrouillage/déverrouillage (110) sont dans la position de déverrouillage intermédiaire, ladite goupille à positionnement radial externe (120) présente une partie extrême fonctionnelle à positionnement radial externe (122) soit exclue du logement traversant (114), soit logée à l'intérieur du logement traversant (114), soit affleurant l'extrémité externe débouchant du logement traversant (114).

4. Conteneur (20) selon la revendication 1, dans lequel le logement traversant (114) et la goupille à positionnement radial externe (120) présentent des longueurs telles que, lorsque les moyens embarqués de verrouillage/déverrouillage (110) sont dans la position de verrouillage final, ladite goupille à positionnement radial externe (120) présente une partie extrême fonctionnelle à positionnement radial externe (122) soit exclue du logement traversant (114), soit logée à l'intérieur du logement traversant (114), soit affleurant l'extrémité externe débouchant du logement traversant (114).

5. Conteneur (20) selon l'une quelconque des revendications 1 à 4, dans lequel la goupille à positionnement radial interne (118) et la goupille à positionnement radial externe (120) présentent des longueurs sensiblement identiques.

6. Conteneur (20) selon l'une quelconque des revendications 1 à 5, dans lequel le logement traversant (114), le logement borgne (116), la goupille à positionnement radial interne (118) et la goupille à positionnement radial externe (120) sont coaxiaux et orientés dans une direction radiale relative à la bride annulaire (30).

7. Conteneur (20) selon l'une quelconque des revendications 1 à 5, dans lequel le logement traversant (114), le logement borgne (116), la goupille à positionnement radial interne et la goupille à positionnement radial externe (120) sont coaxiaux et orientés dans une direction formant un angle α par rapport à une direction radiale relative à la bride annulaire (30).

8. Conteneur (20) selon l'une quelconque des revendications 1 à 7, comprenant des moyens embarqués de cloisonnement et de protection (124) agencés sur un pourtour périphérique externe de la bride annulaire (30) de manière à faire obstacle à la manipulation inopinée des moyens embarqués de verrouillage/déverrouillage (110), **dans lequel de préférence** les moyens embarqués de cloisonnement et de protection (124) comprennent au moins un agencement fonctionnel embarqué de cloisonnement et de protection (126) disposé autour du logement traversant (114) de manière à faire obstacle à la manipulation inopinée de la goupille à positionnement radial externe (120), **dans lequel de préférence** l'agencement fonctionnel embarqué de cloisonnement et de protection (126) présente des dimensions radiales telles que, en position de verrouillage provisoire, la goupille à positionnement radial externe (120) ne s'étend pas au-delà desdits moyens de cloisonnement et de protection (124), **dans lequel de préférence** l'agencement fonctionnel embarqué de cloisonnement et de protection (126) comporte au moins un ergot (128), de préférence deux ergots (128), disposé(s) autour du logement traversant (114) de la bride annulaire (30), **dans lequel de préférence** l'agencement de cloisonnement et de protection (126) comporte un alésage formé dans la bride annulaire (30) et présentant un diamètre supérieur au diamètre du logement traversant (114).

9. Conteneur (20) selon l'une quelconque des revendications 1 à 8, comprenant des moyens embarqués de bridage temporaire (100) agencés sur un pourtour périphérique externe de la bride annulaire (30) de manière à permettre le bridage temporaire du conteneur (20) par blocage axial sur l'enceinte (10) close via l'actionnement du dispositif de jonction étanche (40), **dans lequel de préférence** les moyens embarqués de bridage temporaire (100) comprennent au moins un agencement fonctionnel embarqué de bridage temporaire (102) formé par au moins un ergot (104), de préférence deux ergots (104), présentant une surface fonctionnelle embarquée de bridage axial (106) apte à venir en appui contre une surface fonctionnelle stationnaire de bridage axial (56) du dispositif de jonction étanche (40) de manière à bloquer axialement le déplacement du conteneur (20) vis-à-vis de l'enceinte (10).

10. Conteneur (20) selon la revendication 9, en ce qu'elle dépend de la revendication 8, dans lequel les moyens embarqués de bridage temporaire (100) sont au moins en partie formés par les moyens de protection et de cloisonnement (124), **dans lequel de préférence** l'agencement fonctionnel embarqué de bridage temporaire (102) et l'agencement fonctionnel embarqué de cloisonnement et de protection (126) sont formés par au moins un ergot (104, 128) commun, et de préférence deux ergots (104, 128) communs.

11. Conteneur (20) selon la revendication 10, dans lequel les moyens embarqués de verrouillage/déverrouillage (110) comportent n agencements fonctionnels embarqués de verrouillage/déverrouillage (112) régulièrement répartis autour de la bride annulaire (30) et du couvercle amovible (28), avec n supérieur ou égal à 1, **dans lequel de préférence** les moyens embarqués de cloisonnement et de protection (124) comprennent n agencements fonctionnels embarqués de cloisonnement et de protection (126) régulièrement répartis autour de la bride annulaire (30) et indexés vis-à-vis des n agencements fonctionnels embarqués de verrouillage/déverrouillage (112), **dans lequel de préférence** les moyens embarqués de bridage temporaire (100) comprennent m agencements fonctionnels embarqués de bridage temporaire (102) régulièrement répartis autour de la bride annulaire (30), avec m supérieur ou égal à 1, **dans lequel de préférence** n est égal à m et les agencements fonctionnels embarqués de bridage temporaire (102) sont indexés vis-à-vis des agencements fonctionnels embarqués de verrouillage/déverrouillage (112).

12. Conteneur (20) selon l'une quelconque des revendications 1 à 11, dans lequel le conteneur (20) est de type à usage unique.

13. Ensemble comprenant une enceinte (10), un dispositif de jonction étanche (40) et un conteneur (20) selon l'une quelconque des revendications 1 à 12, en vue de réaliser le transfert aseptique d'un produit biopharmaceutique entre l'enceinte (10) et le conteneur (20).

14. Ensemble selon la revendication 13, dans lequel l'enceinte (10) comprend une paroi périphérique dans laquelle est aménagée une ouverture hermétique obturée par une porte amovible et dans lequel le dispositif de jonction étanche (40) comprend :
des moyens stationnaires de bridage temporaire (50) aptes à maintenir le conteneur (20) bridé contre l'enceinte (10) de sorte que le couvercle amovible (28) dudit conteneur (20) se trouve appliqué hermétiquement contre la porte amovible (18) de ladite enceinte (10) ;
des moyens stationnaires de déverrouillage (60) aptes à faire passer le conteneur (20) d'une position de verrouillage initial dans laquelle le couvercle amovible (28) obture hermétiquement le conteneur (20) à une position de déverrouillage intermédiaire dans laquelle le couvercle amovible (28) est désolidarisé du conteneur (20) et maintenu hermétiquement contre la porte de l'enceinte (10) de manière à assurer une communication aseptique entre ledit conteneur (20) et ladite enceinte (10) ;
des moyens stationnaires de verrouillage (70) aptes à faire passer le conteneur (20) de la position de déverrouillage intermédiaire à une position de verrouillage final dans laquelle ledit couvercle amovible (28) obture à nouveau de façon hermétique le conteneur (20) ;
une couronne fonctionnelle annulaire (42) apte à se déplacer par rotation autour d'un axe géométrique de rotation (R) de manière à actionner les moyens stationnaires de déverrouillage (60) et les moyens stationnaires de verrouillage (70) du conteneur (20) ;
les moyens stationnaires de bridage temporaire (50), les moyens stationnaires de déverrouillage (60) et les moyens stationnaires de verrouillage (70) étant liés mécaniquement à la couronne fonctionnelle annulaire (42) de sorte que la rotation unidirectionnelle de ladite couronne fonctionnelle annulaire (42) autour de l'axe géométrique de rotation (R) entraîne successivement l'actionnement des moyens stationnaires de bridage temporaire (50) assurant le maintien en position du conteneur (20) contre l'enceinte (10), puis l'actionnement des moyens stationnaires de déverrouillage (60) assurant le passage en position de déverrouillage intermédiaire du conteneur (20), puis l'actionnement des moyens stationnaires de verrouillage (70) du conteneur (20) assurant le passage en position de verrouillage final du conteneur (20), et l'actionnement des moyens stationnaires de bridage temporaire (50) du conteneur (20) assurant la libération du conteneur (20).

15. Procédé de transfert aseptique destiné à assurer le transfert aseptique d'un produit biopharmaceutique entre un conteneur (20) et une enceinte (10) appartenant à un ensemble selon l'une des revendications 13 et 14, **caractérisé en ce qu'**il comprend des étapes successives consistant à :
disposer de l'enceinte (10), du dispositif de jonction (40) étanche et du conteneur (20) ; positionner le conteneur (20) contre la paroi périphérique (12) de l'enceinte (10) ;
générer un bridage axial de la bride annulaire (30) du conteneur (20) contre la paroi périphérique (12) de l'enceinte (10) par rotation unidirectionnelle de la couronne fonctionnelle annulaire (42) ;
générer le passage du conteneur (20) de la position de verrouillage initial à la position de déverrouillage intermédiaire par rotation unidirectionnelle de la couronne fonctionnelle annulaire (42) entraînant le déplacement des goupilles à positionnement radial interne (118) et externe (120) à l'intérieur des logements traversant (114) et borgne (116) ;
ouvrir simultanément la porte amovible (18) de l'enceinte (10) et le couvercle amovible (28) du conteneur (20), le couvercle amovible (28) étant fixé hermétiquement contre la porte amovible (18) ; transférer de manière aseptique un ou plusieurs(s) produit(s) biopharmaceutique(s) entre le conteneur (20) et l'enceinte (10) ;
refermer simultanément la porte amovible (18) de l'enceinte (10) et le couvercle amovible (28) (28) du conteneur (20), le couvercle amovible (28) étant fixé hermétiquement contre la porte amovible (18) ;
générer le passage du conteneur (20) par de la position de déverrouillage intermédiaire à la position de verrouillage final par rotation unidirectionnelle de la couronne fonctionnelle annulaire (42) entraînant le déplacement des goupilles à positionnement radial interne (118) et externe (120) à l'intérieur des logements traversant (114) et borgne (116) ;
générer le débridage axial de la bride annulaire (30) du conteneur (20) vis-à-vis de la paroi périphérique (12) de l'enceinte (10) par rotation unidirectionnelle de la couronne fonctionnelle annulaire (42).

## Patentansprüche

1. Behälter (20), insbesondere zur Gewährleistung des Transports und des aseptischen Transfers eines biopharmazeutischen Produkts aus einer oder in eine geschlossene(n) Kammer (10), die mit einer Vorrichtung (40) zur dichten Verbindung versehen ist, umfassend:
einen ringförmigen Flansch (30), der eine Öffnung (26) begrenzt;
einen abnehmbaren Deckel (28), der die Öffnung (26) des ringförmigen Flansches (30) hermetisch verschließen kann;
eingebaute Mittel (110) zur Verriegelung/Entriegelung des abnehmbaren Deckels (28) auf dem ringförmigen Flansch (30); und
einen Umfangsmantel (22), der fest mit dem ringförmigen Flansch (30) verbunden ist und einen eingeschlossenen Innenraum (24) zur Aufnahme von zu dem biopharmazeutischen Bereich gehörenden Produkten begrenzt;
wobei die eingebauten Verriegelungs-/Entriegelungsmittel (110) mindestens eine eingebaute funktionale Verriegelungs-/Entriegelungsanordnung (112) umfassen, die einerseits durch eine in dem ringförmigen Flansch (30) ausgebildete Durchgangsaufnahme (114) und eine Sackaufnahme (116), die in dem abnehmbaren Deckel (28) und in der Verlängerung der Durchgangsaufnahme (114) ausgebildet ist, wenn der abnehmbare Deckel (28) die Öffnung des ringförmigen Flansches (30) hermetisch verschließt, und andererseits durch einen radial innen positionierbaren Stift (118) und einen radial außen positionierbaren Stift (120), die in die Sackaufnahme (116) des abnehmbaren Deckels (28) und die Durchgangsaufnahme (114) des ringförmigen Flansches (30) eingeführt werden können und sich darin bewegen können, gebildet wird;
wobei sich der Behälter (20) in einer anfänglichen Verriegelungsposition befinden kann, in der einerseits der radial innen positionierbare Stift (118) einen funktionalen inneren Teil (118INT) zur anfänglichen Verriegelung, der in der Sackaufnahme (116) des abnehmbaren Deckels (28) angeordnet ist, und einen funktionalen äußeren Teil (118EXT) zur anfänglichen Verriegelung, der in der Durchgangsaufnahme (114) des ringförmigen Flansches (30) angeordnet ist, aufweist, um die relative Bewegung des abnehmbaren Deckels (28) bezüglich des ringförmigen Flansches (30) zu verhindern, und andererseits der radial außen positionierbare Stift (120) zumindest teilweise in der Durchgangsaufnahme (114) des ringförmigen Flansches (30) angeordnet ist;
wobei sich der Behälter (20) in einer Zwischenentriegelungsposition befinden kann, in der einerseits der radial innen positionierbare Stift (118) zumindest teilweise in der Sackaufnahme (116) des abnehmbaren Deckels (28) und vollständig außerhalb der Durchgangsaufnahme (114) des ringförmigen Flansches (30) angeordnet ist, und andererseits der radial außen positionierbare Stift (120) zumindest teilweise in der Durchgangsaufnahme (114) des ringförmigen Flansches (30) und vollständig außerhalb der Sackaufnahme (116) des abnehmbaren Deckels (28) angeordnet ist, um die relative Bewegung des abnehmbaren Deckels (28) bezüglich des ringförmigen Flansches (30) zu gestatten;
**dadurch gekennzeichnet, dass** sich der Behälter (20) in einer Endverriegelungsposition befinden kann, in der einerseits der radial innen positionierbare Stift (118) in der Sackaufnahme (116) des abnehmbaren Deckels (28) angeordnet ist und andererseits der radial außen positionierbare Stift (120) einen funktionalen inneren Teil (120INT) zur Endverriegelung, der in der Sackaufnahme (116) des abnehmbaren Deckels (28) angeordnet ist, und einen funktionalen äußeren Teil (120EXT) zur Endverriegelung, der in der Durchgangsaufnahme (114) des ringförmigen Flansches (30) angeordnet ist, aufweist, um die relative Bewegung des abnehmbaren Deckels (28) bezüglich des ringförmigen Flansches (30) zu verhindern.

2. Behälter (20) nach Anspruch 1, wobei die Durchgangsaufnahme (114) und der radial außen positionierbare Stift (120) eine solche Länge aufweisen, dass, wenn sich die eingebauten Verriegelungs-/Entriegelungsmittel (110) in der anfänglichen Verriegelungsposition befinden, der radial außen positionierbare Stift (120) einen radial außen positionierbaren funktionalen Endteil (122) aufweist, der sich entweder außerhalb der Durchgangsaufnahme (114) befindet oder innerhalb der Durchgangsaufnahme (114) aufgenommen ist oder bündig mit dem in der Durchgangsaufnahme (114) mündenden äußeren Ende ist.

3. Behälter (20) nach Anspruch 1, wobei die Durchgangsaufnahme (114) und der radial außen positionierbare Stift (120) eine solche Länge aufweisen, dass, wenn sich die eingebauten Verriegelungs-/Entriegelungsmittel (110) in der Zwischenentriegelungsposition befinden, der radial außen positionierbare Stift (120) einen radial außen positionierbaren funktionalen Endteil (122) aufweist, der sich entweder außerhalb der Durchgangsaufnahme (114) befindet oder innerhalb der Durchgangsaufnahme (114) aufgenommen ist oder bündig mit dem in der Durchgangsaufnahme (114) mündenden äußeren Ende ist.

4. Behälter (20) nach Anspruch 1, wobei die Durchgangsaufnahme (114) und der radial außen positionierbare Stift (120) eine solche Länge aufweisen, dass, wenn sich die eingebauten Verriegelungs-/Entriegelungsmittel (110) in der Endverriegelungsposition befinden, der radial außen positionierbare Stift (120) einen radial außen positionierbaren funktionalen Endteil (122) aufweist, der sich entweder außerhalb der Durchgangsaufnahme (114) befindet oder innerhalb der Durchgangsaufnahme (114) aufgenommen ist oder bündig mit dem in der Durchgangsaufnahme (114) mündenden äußeren Ende ist.

5. Behälter (20) nach einem der Ansprüche 1 bis 4, wobei der radial innen positionierbare Stift (118) und der radial außen positionierbare Stift (120) eine im Wesentlichen identische Länge aufweisen.

6. Behälter (20) nach einem der Ansprüche 1 bis 5, wobei die Durchgangsaufnahme (114), die Sackaufnahme (116), der radial innen positionierbare Stift und der radial außen positionierbare Stift (120) koaxial sind und in einer radialen Richtung bezüglich des ringförmigen Flansches (30) ausgerichtet sind.

7. Behälter (20) nach einem der Ansprüche 1 bis 5, wobei die Durchgangsaufnahme (114), die Sackaufnahme (116), der radial innen positionierbare Stift und der radial außen positionierbare Stift (120) koaxial sind und in einer bezüglich einer bezüglich des ringförmigen Flansches (30) radialen Richtung einen Winkel α bildenden Richtung ausgerichtet sind.

8. Behälter (20) nach einem der Ansprüche 1 bis 7, der eingebaute Trenn- und Schutzmittel (124) umfasst, die auf einem Außenumfangsrand des ringförmigen Flansches (30) angeordnet sind, um eine versehentliche Betätigung der eingebauten Verriegelungs-/Entriegelungsmittel (110) zu behindern, wobei die eingebauten Trenn- und Schutzmittel (124) vorzugsweise mindestens eine eingebaute funktionale Trenn- und Schutzanordnung (126) umfassen, die um die Durchgangsaufnahme (114) herum angeordnet ist, um die versehentliche Betätigung des radial außen positionierbaren Stifts (120) zu behindern, wobei die eingebaute funktionale Trenn- und Schutzanordnung (126) vorzugsweise solche Radialabmessungen aufweist, dass sich der radial außen positionierbare Stift (120) in der vorübergehenden Verriegelungsposition nicht über die Trenn- und Schutzmittel (124) hinaus erstreckt, wobei die eingebaute funktionale Trenn- und Schutzanordnung (126) vorzugsweise mindestens eine Nase (128), vorzugsweise zwei Nasen (128) umfasst, die um die Durchgangsaufnahme (114) des ringförmigen Flansches (30) herum angeordnet ist/sind, wobei die Trenn- und Schutzanordnung (126) vorzugsweise eine Bohrung umfasst, die in dem ringförmigen Flansch (30) ausgebildet ist und einen Durchmesser aufweist, der größer als der Durchmesser der Durchgangsaufnahme (114) ist.

9. Behälter (20) nach einem der Ansprüche 1 bis 8, der eingebaute Mittel (100) zum vorübergehenden Einklemmen umfasst, die auf dem Außenumfangsrand des ringförmigen Flansches (30) angeordnet sind, um das vorübergehende Einklemmen des Behälters (20) durch Blockieren in Axialrichtung an der geschlossenen Kammer (10) über die Betätigung der Vorrichtung (40) zur dichten Verbindung zu gestatten, wobei die eingebauten Mittel (100) zum vorübergehenden Einklemmen vorzugsweise mindestens eine durch mindestens eine Nase (104), vorzugsweise zwei Nasen (104), gebildete eingebaute funktionale Anordnung (102) zum vorübergehenden Einklemmen umfassen, die eine eingebaute funktionale Fläche (106) zum axialen Einklemmen aufweist, die an eine funktionale stationäre Fläche (56) zum axialen Einklemmen der Vorrichtung (40) zur dichten Verbindung zur Anlage kommen kann, um die Bewegung des Behälters (20) bezüglich der Kammer (10) in Axialrichtung zu blockieren.

10. Behälter (20) nach Anspruch 9, sofern von Anspruch 8 abhängig, wobei die eingebauten Mittel (100) zum vorübergehenden Einklemmen zumindest teilweise durch die Trenn- und Schutzmittel (124) gebildet werden, wobei die eingebaute funktionale Anordnung (102) zum vorübergehenden Einklemmen und die eingebaute funktionale Trenn- und Schutzanordnung (126) vorzugsweise durch mindestens eine gemeinsame Nase (104, 128) und vorzugsweise zwei gemeinsame Nasen (104, 128) gebildet werden.

11. Behälter (20) nach Anspruch 10, wobei die eingebauten Verriegelungs-/Entriegelungsmittel (110) n eingebaute funktionale Verriegelungs-/Entriegelungsanordnungen (112) umfassen, die gleichmäßig um den ringförmigen Flansch (30) und den abnehmbaren Deckel (28) herum verteilt sind, wobei n größer gleich 1 ist, wobei die eingebauten Trenn- und Schutzmittel (124) vorzugsweise n eingebaute funktionale Trenn- und Schutzanordnungen (126) umfassen, die gleichmäßig um den ringförmigen Flansch (30) herum verteilt sind und bezüglich der n eingebauten funktionalen Verriegelungs-/Entriegelungsanordnungen (112) fixiert sind, wobei die eingebauten Mittel (100) zum vorübergehenden Einklemmen vorzugsweise m eingebaute funktionale Anordnungen (102) zum vorübergehenden Einklemmen umfassen, die gleichmäßig um den ringförmigen Flansch (30) herum verteilt sind, wobei m größer gleich 1 ist, wobei n vorzugsweise gleich m ist und die eingebauten funktionalen Anordnungen (102) zum vorübergehenden Einklemmen bezüglich der eingebauten funktionalen Verriegelungs-/Entriegelungsnordnungen (112) fixiert sind.

12. Behälter (20) nach einem der Ansprüche 1 bis 11, wobei der Behälter (20) jener Art für den einmaligen Gebrauch ist.

13. Einheit, die eine Kammer (10), eine Vorrichtung (40) zur dichten Verbindung und einen Behälter (20) nach einem der Ansprüche 1 bis 12 zur Durchführung des aseptischen Transfers eines biopharmazeutischen Produkts zwischen der Kammer (10) und dem Behälter (20) umfasst.

14. Einheit nach Anspruch 13, wobei die Kammer (10) eine Umfangswand umfasst, in der eine durch eine abnehmbare Tür hermetisch verschlossene Öffnung ausgebildet ist, und wobei die Vorrichtung (40) zur dichten Verbindung Folgendes umfasst:
stationäre Mittel (50) zum vorübergehenden Einklemmen, die den Behälter (20) gegen die Kammer (10) geklemmt halten können, so dass der abnehmbare Deckel (28) des Behälters (20) hermetisch dicht gegen die abnehmbare Tür (18) der Kammer (10) gedrückt wird;
stationäre Mittel (60) zum Entriegeln, die den Behälter (20) aus einer anfänglichen Verriegelungsposition, in der der abnehmbare Deckel (28) den Behälter (20) hermetisch verschließt, in eine Zwischenentriegelungsposition, in der der abnehmbare Deckel (28) von dem Behälter (20) getrennt ist und hermetisch dicht gegen die Tür der Kammer (10) gehalten wird, um eine aseptische Verbindung zwischen dem Behälter (20) und der Kammer (10) zu gewährleisten, führen können;
stationäre Verriegelungsmittel (70), die den Behälter (20) aus der Zwischenentriegelungsposition in eine Endverriegelungsposition, in der der abnehmbare Deckel (28) den Behälter (20) erneut hermetisch verschließt, führen können;
einen funktionalen ringförmigen Kranz (42), der sich um eine geometrische Drehachse (R) drehen kann, um die stationären Entriegelungsmittel (60) und die stationären Verriegelungsmittel (70) des Behälters (20) zu betätigen;
wobei die stationären Mittel (50) zum vorübergehenden Einklemmen, die stationären Entriegelungsmittel (60) und die stationären Verriegelungsmittel (70) mechanisch so mit dem funktionalen ringförmigen Kranz (42) verbunden sind, dass die unidirektionale Drehung des funktionalen ringförmigen Kranzes (42) um die geometrische Drehachse (R) nacheinander die Betätigung der stationären Mittel (50) zum vorübergehenden Einklemmen, die das Festhalten des Behälters (20) gegen die Kammer (10) gewährleisten, dann die Betätigung der stationären Entriegelungsmittel (60), die das Führen des Behälters (20) in die Zwischenentriegelungsposition gewährleisten, dann die Betätigung der stationären Verriegelungsmittel (70) des Behälters (20), die das Führen des Behälters (20) in die Endverriegelungsposition gewährleisten, und die Betätigung der stationären Mittel (50) zum vorübergehenden Einklemmen des Behälters (20), die die Freigabe des Behälters (20) gewährleisten, bewirkt.

15. Verfahren zum aseptischen Transfer zur Gewährleistung des aseptischen Transfers eines biopharmazeutischen Produkts zwischen einem Behälter (20) und einer Kammer (10), die zu einer Einheit nach einem der Ansprüche 13 und 14 gehören, **dadurch gekennzeichnet, dass** es die folgenden aufeinanderfolgenden Schritte umfasst:
unter Bereitstellung der Kammer (10), der Vorrichtung (40) zur dichten Verbindung und des Behälters (20) Positionieren des Behälters (20) gegen die Umfangswand (12) der Kammer (10);
Hervorrufen eines axialen Einklemmens des ringförmigen Flansches (30) des Behälters (20) gegen die Umfangswand (12) der Kammer (10) durch unidirektionales Drehen des funktionalen ringförmigen Kranzes (42);
Hervorrufen des Führens des Behälters (20) aus der anfänglichen Verriegelungsposition in die Zwischenentriegelungsposition durch unidirektionales Drehen des funktionalen ringförmigen Kranzes (42), wodurch die Bewegung des radial innen (118) und außen (120) positionierbaren Stifts im Inneren der Durchgangsaufnahme (114) und Sackaufnahme (116) bewirkt wird;
gleichzeitiges Öffnen der abnehmbaren Tür (18) der Kammer (10) und des abnehmbaren Deckels (28) des Behälters (20), wobei der abnehmbare Deckel (28) hermetisch gegen die abnehmbare Tür (18) befestigt ist;
aseptisches Transferieren eines oder mehrerer biopharmazeutischer Produkte zwischen dem Behälter (20) und der Kammer (10);
gleichzeitiges Schließen der abnehmbaren Tür (18) der Kammer (10) und des abnehmbaren Deckels (28) des Behälters (20), wobei der abnehmbare Deckel (28) hermetisch gegen die abnehmbare Tür (18) befestigt ist;
Hervorrufen des Führens des Behälters (20) aus der Zwischenentriegelungsposition in die Endverriegelungsposition durch unidirektionales Drehen des funktionalen ringförmigen Kranzes (42), wodurch die Bewegung des radial innen (118) und außen (120) positionierbaren Stifts im Inneren der Durchgangsaufnahme (114) und Sackaufnahme (116) bewirkt wird;
Hervorrufen des axialen Lösens des ringförmigen Flansches (30) des Behälters (20) bezüglich der Umfangswand (12) der Kammer (10) durch unidirektionales Drehen des funktionalen ringförmigen Kranzes (42).

## Claims

1. Container (20) specially intended for the transport and aseptic transfer of a biopharmaceutical product from or to a closed chamber (10) equipped with a leaktight joining device (40), comprising :
an annular flange (30) delimiting an opening (26);
a removable cover (28) designed to hermetically seal the opening (26) of the annular flange (30);
integrated means (110) for locking/unlocking (110) the removable cover (28) on the annular flange (30); and
a peripheral envelope (22) integral with the annular flange (30) and delimiting a confined inside space (24) designed for receiving products belonging to the biopharmaceutical field;
wherein the integrated locking/unlocking means (110) comprise at least one integrated functional locking/unlocking arrangement (112) formed, on the one hand, by a through-housing (114) provided in the annular flange (30) and a blind housing (116) provided in the removable cover (28) and in the extension of the through-housing (114) when the removable cover (28) is hermetically sealing the opening of the annular flange (30) and, on the other hand, by a pin (118) at a radially inner position and a pin (120) at a radially outer position which can be introduced into and moved inside the blind housing (116) of the removable cover (28) and the through-housing (114) of the annular flange (30);
the container (20) being such that it can be disposed in an initial locking position in which, on the one hand, the pin (118) at a radially inner position has an internal functional portion (118_{INT}) for initial locking disposed in the blind housing (116) of the removable cover (28) and an external functional portion (118_{EXT}) for initial locking disposed in the through-housing (114) of the annular flange (30) in order to prevent the relative movement of the removable cover (28) with respect to the annular flange (30) and, on the other hand, the pin (120) at a radially outer position (120) is at least partially disposed in the through-housing (114) of the annular flange (30);
the container (20) being such that it can be disposed in an intermediate unlocking position in which, on the one hand, the pin (118) at the radially inner position (118) is at least partially disposed in the blind housing (116) of the removable cover (28) and completely outside the through-housing (114) of the annular flange (30) and, on the other hand, the pin (120) at the radially outer position is at least partially disposed in the through-housing (114) of the annular flange (30) and completely outside the blind housing (116) of the removable cover (28) so as to allow the relative movement of the removable cover (28) with respect to the annular flange (30);
**characterised in that** the container (20) is such that it can be disposed in a final locking position in which, on the one hand, the pin at the radially inner position (118) is disposed in the blind housing (116) of the removable cover (28) and, on the other hand, the pin at the radially outer position (120) has an internal functional portion (120_{INT}) for final locking disposed in the blind housing (116) of the removable cover (28) and an external functional portion (120_{EXT}) for final locking disposed in the through-housing (114) of the annular flange (30) in order to prevent the relative movement of the removable cover (28) with respect to the annular flange (30).

2. Container (20) as claimed in claim 1, wherein the through-housing (114) and the pin (120) at the radially outer position have lengths such that when the integrated locking/unlocking means (110) are in the initial locking position, said pin (120) at the radially outer position has a functional end part (122) at the radially outer position that is either outside the through-housing (114) or is housed inside the through-housing (114) or is flush with the open outer end of the through-housing (114).

3. Container (20) as claimed in claim 1, wherein the through-housing (114) and the pin (120) at the radially outer position have lengths such that when the integrated locking/unlocking means (110) are in the intermediate unlocking position, said pin (120) at the radially outer position has a functional end part (122) at the radially outer position that is either outside the through-housing (114) or housed inside the through-housing (114) or flush with the open outer end of the through-housing (114).

4. Container (20) as claimed in claim 1, wherein the through-housing (114) and the pin (120) at the radially outer position have lengths such that when the integrated locking/unlocking means (110) are in the final locking position, said pin (120) at the radially outer position has a functional end part (122) at the radially outer position that is either outside the through-housing (114) or housed inside the through-housing (114) or flush with the open outer end of the through-housing (114).

5. Container (20) as claimed in any one of claims 1 to 4, wherein the pin (118) at the radially inner position and the pin (120) at the radially outer position have substantially identical lengths.

6. Container (20) as claimed in any one of claims 1 to 5, wherein the through-housing (114), the blind housing (116), the pin (118) at the radially inner position and the pin (120) at the radially outer position (120) are coaxial and oriented in a radial direction relative to the annular flange (30).

7. Container (20) as claimed in any one of claims 1 to 5, wherein the through-housing (114), the blind housing (116), the pin (118) at the radially inner position and the pin (120) at the radially outer position are coaxial and oriented in a direction subtending an angle α with a radial direction relative to the annular flange (30).

8. Container (20) as claimed in any one of claims 1 to 7, comprising integrated isolation and protection means (124) disposed on an outer peripheral edge of the annular flange (30) in order to obstruct inadvertent manipulation of the integrated locking/unlocking means (110), **wherein by preference** the integrated isolation and protection means (124) comprise at least one integrated functional isolation and protection arrangement (126) disposed around the through-housing (114) in order to obstruct inadvertent manipulation of the pin (120) at the radially outer position, **wherein by preference** the integrated functional isolation and protection arrangement (126) has radial dimensions such that in a temporary locking position, the (120) pin at the radially outer position does not extend beyond said isolation and protection means (124), **wherein by preference** the integrated functional isolation and protection arrangement (126) comprises at least one lug (128), preferably two lugs (128), disposed around the through-housing (114) of the annular flange (30), **wherein by preference** the isolation and protection arrangement (126) comprises a bore provided in the annular flange (30) and having a diameter bigger than the diameter of the through-housing (114).

9. Container (20) as claimed in any one of claims 1 to 8, comprising integrated temporary clamping means (100) disposed on an outer peripheral edge of the annular flange (30) to enable the container (20) to be temporarily clamped on the closed chamber (10) by an axial locking action by actuating the leaktight joining device (40), wherein by preference the integrated temporary clamping means (100) comprise at least one integrated functional arrangement for temporary clamping (102) provided in the form of at least one lug (104), preferably two lugs (104), having an integrated functional axial clamping surface (106) designed to move into abutment with a stationary functional axial clamping surface (56) of the leaktight joining device (40) in order to prevent axial movement of the container (20) relative to the chamber (10).

10. Container (20) as claimed in claim 9, relating back to claim 8, wherein the integrated temporary clamping means (100) are at least partly formed by the isolation and protection means (124), **wherein by preference** the integrated functional arrangement for temporary clamping (102) and the integrated functional isolation and protection arrangement (126) are formed by at least one common lug (104, 128) and preferably two common lugs (104, 128).

11. Container (20) as claimed in claim 10, wherein the integrated locking/unlocking means (110) comprise n integrated functional locking/unlocking arrangements (112) regularly distributed around the annular flange (30) and the removable cover (28), where n is greater than or equal to 1, **wherein by preference** the integrated isolation and protection means (124) comprise n integrated functional isolation and protection arrangements (126) regularly distributed around the annular flange (30) and indexed relative to the n integrated functional locking/unlocking arrangements (112), **wherein by preference** the integrated temporary clamping means (100) comprise m integrated functional temporary clamping arrangements (102) regularly distributed around the annular flange (30), where m is greater than or equal to 1, **wherein by preference** n is equal to m and the integrated functional temporary clamping arrangements (102) are indexed relative to the integrated functional locking/unlocking arrangements (112).

12. Container (20) as claimed in any one of claims 1 to 11, wherein the container (20) is of the disposable type.

13. Assembly comprising a chamber (10), a leaktight joining device (40) and a container (20) as claimed in any one of claims 1 to 12, for making an aseptic transfer of a biopharmaceutical product between the chamber (10) and the container (20).

14. Assembly as claimed in claim 13, wherein the chamber (10) comprises a peripheral wall in which an opening is provided, hermetically sealed by a removable door, and wherein the leaktight joining device (40) comprises :
stationary temporary clamping means (50) designed to hold the container (20) clamped against the chamber (10) so that the removable cover (28) of said container (20) is hermetically applied against the removable door (18) of said chamber (10);
stationary unlocking means (60) designed to enable the container (20) to be switched from an initial locking position in which the removable cover (28) hermetically seals the container (20) into an intermediate unlocking position in which the removable cover (28) is detached from the container (20) and held in a hermetically sealing arrangement against the door of the chamber (10) in order to ensure an aseptic communication between said container (20) and said chamber (10);
stationary locking means (70) designed to enable the container (20) to be switched from the intermediate unlocking position into a final locking position in which said removable cover (28) hermetically seals the container (20) again;
an annular functional ring gear (42) designed to rotate about a geometric axis of rotation (R) in order to actuate the stationary unlocking means (60) and the stationary locking means (70) of the container (20);
the stationary temporary clamping means (50), the stationary unlocking means (60) and the stationary locking means (70) being mechanically connected to the annular functional ring gear (42) so that a one-way rotation of said annular functional ring gear (42) about the geometric axis of rotation (R) successively drives actuation of the stationary temporary clamping means (50) in order to hold the container (20) in position against the chamber (10), then actuate the stationary unlocking means (60) in order to switch to the intermediate unlocking position of the container (20), then actuate the stationary locking means (70) of the container (20) to enable the container (20) to be switched to the final locking position and actuate the stationary temporary clamping means (50) of the container (20) in order to release the container (20).

15. Method of making am aseptic transfer intended for the aseptic transfer of a biopharmaceutical product between a container (20) and a chamber (10) belonging to an assembly as claimed in one of claims 13 and 14, **characterised in that** it comprises successive steps involving:
taking the chamber (10), the leaktight joining device (40) and the container (20);
positioning the container (20) against the peripheral wall (12) of the chamber (10);
initiating axial clamping of the annular flange (30) of the container (20) against the peripheral wall (12) of the chamber (10) by a one-way rotation of the annular functional ring gear (42);
initiating switching of the container (20) from the initial locking position into the intermediate unlocking position by a one-way rotation of the annular functional ring gear (42) in order to drive the movement of pins at a radially inner (118) and outer (120) position inside the through-housing (114) and blind housing (116);
simultaneously opening the removable door (18) of the chamber (10) and the removable cover (28) of the container (20), the removable cover (28) being hermetically secured against the removable door (18);
aseptically transferring one or more biopharmaceutical product(s) between the container (20) and the chamber (10);
simultaneously reclosing the removable door (18) of the chamber (10) and the removable cover (28) of the container (20), the removable cover (28) being hermetically secured against the removable door (18);
initiating the switch of the container (20) via the intermediate unlocking position into the final locking position by a one-way rotation of the annular functional ring gear (42) in order to drive the movement of pins at a radially outer (118) and inner (120) position inside a through-housing (114) and blind housing (116) ;
initiating axial unclamping of the annular flange (30) of the container (20) with respect to the peripheral wall (12) of the chamber (10) by a one-way rotation of the annular functional ring gear (42).
